# EUROPEAN PATENT SPECIFICATION

(11) **EP 3 372 596 B1**
(45) Date of publication and mention of the grant of the patent: **22.07.2020**
(21) Application number: 16861553.2
(22) Date of filing: 02.11.2016
(51) Int. Cl.: C07D 413/14, C07D 401/04

(54) **PREPARATION METHOD FOR TEDIZOLID, TEDIZOLID INTERMEDIATE, AND PREPARATION METHOD THEREFOR**
VERFAHREN ZUR HERSTELLUNG VON TEDIZOLID, TEDIZOLIDZWISCHENPRODUKT UND HERSTELLUNGSVERFAHREN DAFÜR
PROCÉDÉ DE PRÉPARATION DE TÉDIZOLIDE, INTERMÉDIAIRE DE TÉDIZOLIDE ET PROCÉDÉ POUR SA PRÉPARATION

(30) Priority: 03.11.2015 CN 201510739910
(43) Date of publication of application: 12.09.2018
(73) Proprietor: Zhejiang Huahai Pharmaceutical Co., Ltd, Zhejiang 317024 (CN); Shanghai Syncores Technologies Inc. Ltd., Shanghai 201203 (CN)
(72) Inventor: LI, Siyuan, Shanghai 201203 (CN); GUI, Shaoxiao, Shanghai 201203 (CN); ZHANG, Jicheng, Shanghai 201203 (CN); HUANG, Luning, Shanghai 201203 (CN); TAO, Anping, Shanghai 201203 (CN); GU, Hong, Shanghai 201203 (CN)
(74) Representative: Zwicker, Jörk
(86) International application number: PCT/CN2016/104311
(87) International publication number: WO 2017/076285

(56) References cited:
- WO-A1-2004/056818
- WO-A1-2005/058886
- WO-A2-2010/042887
- CN-A- 104 327 119
- CN-A- 104 496 979
- CN-A- 104 892 592
- DATABASE CA [Online] CHEMICAL ABSTRACTS SERVICE, COLUMBUS, OHIO, US; 26 August 2016 (2016-08-26), DESI REDDY, SRINIVAS REDDY ET AL.: "A novel process for the preparation of Tedizolid", XP002789889, retrieved from STN Database accession no. 2016:1450816 -& DESI REDDY, SRINIVAS REDDY ET AL.: "IN2014CH05297: A novel process for the preparation of Tedizolid", , 26 August 2016 (2016-08-26), pages 1-25, XP055571801, Retrieved from the Internet: URL:https://content2.cas.org/v1/AUTH_9a355 bb5cefd4c378bde0d541c6a11ce/patentpak-cdr- full-text-47/patent/76871682_1537160410.pd f?temp_url_sig=64205b179b98db80e074b67be1b 5c5b1de91ae25&temp_url_expires=1553107130& inline [retrieved on 2019-03-20]

## Description

### TECHNICAL FIELD

The invention relates to a preparation method for a novel oxazolidinone antibiotic tedizolid or its phosphates, and its intermediate compounds and the preparation method thereof.

### BACKGROUND ART

Tadizolid phosphate has a strong antibacterial activity to pathogens of human and animal, including gram-positive bacteria such as *Staphylococcus, Enterococcus* and *Streptococcus,* anaerobic microorganisms such as bacteroid and *Clostridium,* and acidotolerant microorganisms such as *Mycobacterium tuberculosis* and *Mycobacterium avium complex.* Tadizolid (formerly called torezolid) was jointly developed by the Cubist Pharmaceuticals company (a subsidiary of Merck Corporation) and Bayer. It was originally found as an antibacterial drug precursor by Dong-A Pharmaceutical (Dong-A ST) and used for the treatment of gram-positive bacterial infections. Tedizolid is rapidly transformed into its active form TR 700 (DA 7157) in plasma.

WO2005058886A1 discloses the synthesis of 3-[3-fluoro-4-[6-(2-methyl-2H- tetrazol-5-yl)-3-pyridinyl]phenyl]-5-(hydroxymethyl)-2-oxazolidinone, wherein 3-fluoroaniline is used as raw material and reacts with glycidyl butyrate after being protected by Cbz to obtain compound 3. Compound 3 is then iodinated and converted into tin reagent 5, which is Suzuki coupled with 5-bromo-2-(2-methyl-2H-tetrazol-5-yl)-pyridine to produce the key intermediate K. The reaction scheme is depicted as follows:

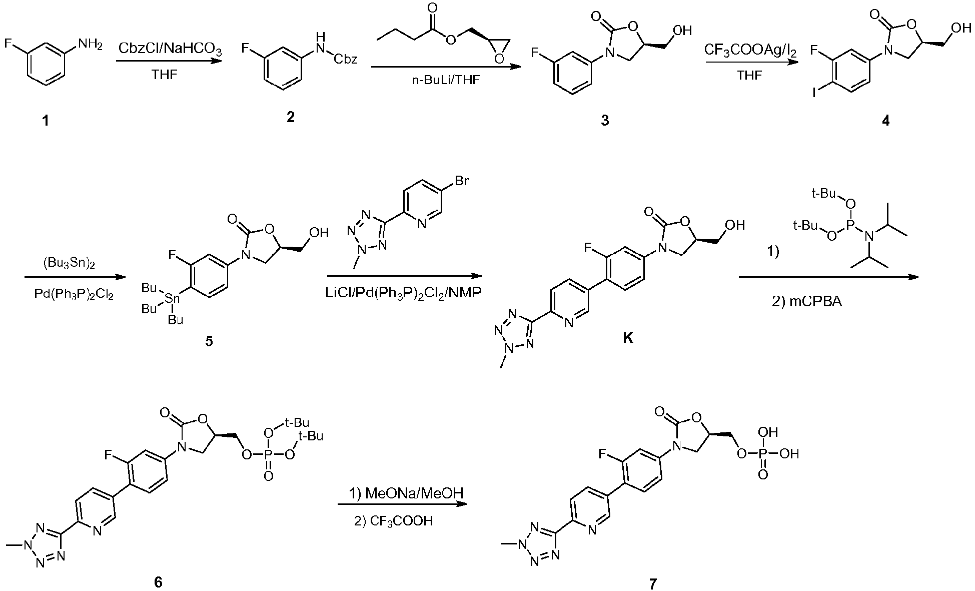

The reaction procedure of the original drug of Dong-A Pharmaceutical is long, and the total yield is not high. In terms of cost, relatively expensive reagents such as CF₃COOAg are needed, and Pd catalyst are needed twice for respectively preparing intermediates 5 and K in the scheme. The reaction conditions are harsh, which is not easy for a large scale production.

Later, the synthetic scheme of the original compound was improved by an licensee Trius Therapeutics company, in whose patent WO2010042887, 4-bromo-3-fluoroaniline is employed as the starting material, first to synthesize boric acid ester 10, which is then Suzuki coupled with 5-bromo-2-(2-methyl-2H- tetrazol-5-yl)-pyridine to generate intermediate 11. Intermediate 11 then reacts with glycidyl butyrate to obtain the oxazolidinone intermediate K. The reaction scheme is depicted as follows:

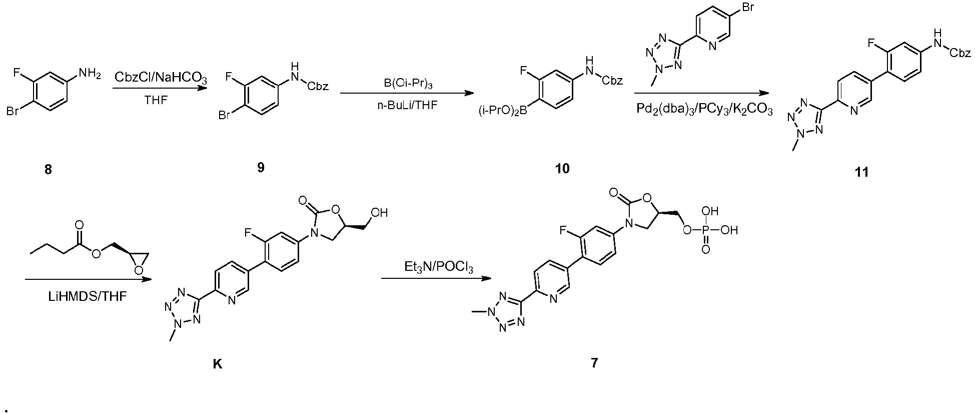

Compared with the patented method of Dong-A Pharmaceutical, this scheme is short in reaction procedure with an improved total yield. However, the reaction conditions are still rather harsh. Butyl lithium is needed, and the reaction needs to be carried out at ultra-low temperature (-65 °C). The use of n-BuLi and LiHMDS requires strict anhydrous condition.

In addition, CN104496979A discloses a method for preparing tedizolid, which is as depicted in the reaction scheme below: 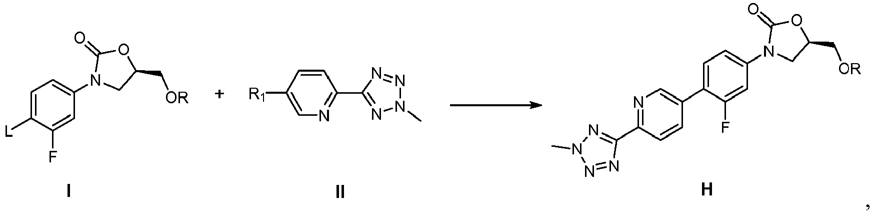 wherein R is hydrogen or a hydroxyl protective group; one of L and R₁ is a leaving group, while the other one is BF₃ or BR₂R₃, wherein R₂ and R₃ are independently selected from a group consisting of OH, and optionally substituted C₁-C₆ monohydric alcohol and C₁-C₆ diol, and wherein R₂ and R₃ can form a ring. Pd is employed in this scheme to catalyze the synthesis of borate intermediate II. After separation and purification, the intermediate II is Suzuki coupled with compound I under catalysis of Pd to obtain the compound of formula H. In this scheme, the reaction operations are complicated, since the intermediate II needs to be separated out before Suzuki coupling.

The methods of prior art for preparing tedizolid intermediates all suffer from complex operation, long reaction time, low total yield and low purity.

### SUMMARY OF THE INVENTION

One purpose of the invention is to provide a preparation method of tedizolid, which has low production cost, simple operation, relatively high yield and high purity, and is suitable for industrial production. In particular, the invention relates to a novel method for preparing tedizolid by using novel intermediates.

To achieve the above purpose, the invention provides a method for preparing a tedizolid compound of the formula below 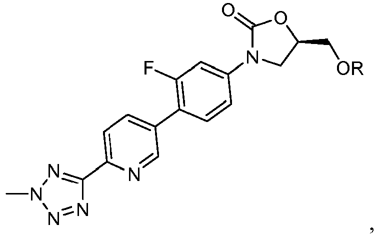
wherein R is selected from a group consisting of hydrogen , 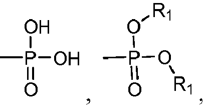 benzyl or benzyl substituted by a substituent (the substituent is selected from a group consisting of halogen, nitro, C₁-C₆ alkyl and C₁-C₆ alkoxy), and R₁ is C₁-C₆ alkyl or C₁-C₆ alkyl substituted by halogen;
wherein the method comprises reacting the compound of the formula below 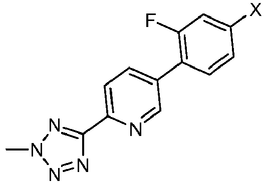
wherein X is chlorine, bromine , iodine, or sulfonyloxy such as trifluoromethane sulfonyloxy, methanesulfonyloxy, benzenesulfonyloxy, or benzenesulfonyloxy substituted by one or more substituents, and the substituents are selected from the group consisting of halogen, C₁-C₆ alkyl and C₁-C₆ alkoxy; preferably, X is chlorine, bromine or iodine; and more preferably, X is bromine or iodine,
with the compound of the formula below 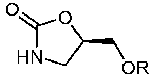 by catalysis of a metal catalyst by a coupled reaction, wherein the substituent R is as defined above.

In a preferred embodiment of the present invention, the metal catalyst is a copper catalyst. The copper catalyst is preferably selected from a group consisting of Cu powder, CuI, CuBr, Cu₂O, CuO, Cu₂O, CuSO₄, Cu(OAc)₂ or Cu(OTf)₂, and more preferably CuI and Cu(OAc)₂.

In addition to using a copper catalyst alone, in some instances, ligands are also required for the reaction. Diamine ligands, diketone ligands, phenanthroline ligands, amino acid ligands, or Phos ligands can be used. The diamine ligands are preferably selected from a group consisting of:

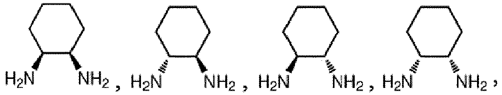

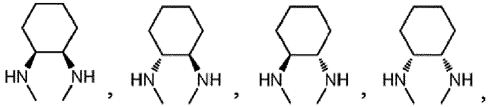

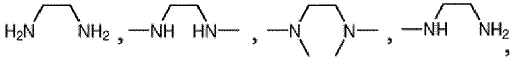

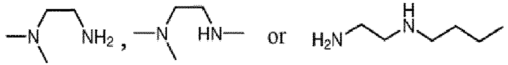

The diketone ligands are preferably selected from a group consisting of:

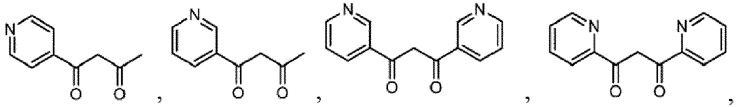

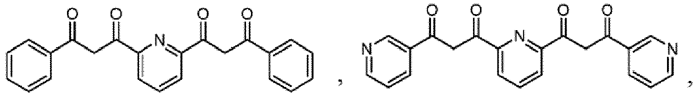

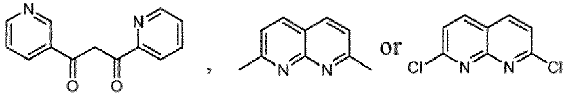

The phenanthroline ligands are preferably selected from a group consisting of:

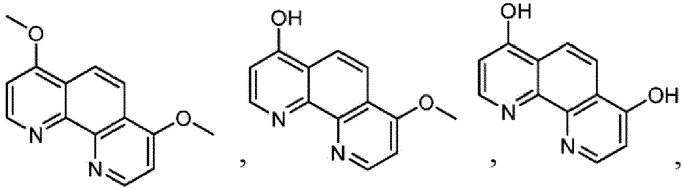

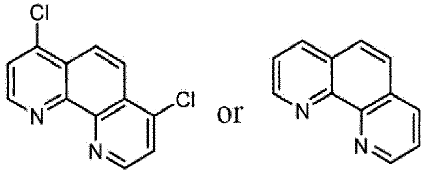

The amino acid ligands are preferably selected from a group consisting of:

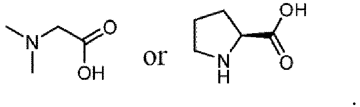

The Phos ligands are preferably selected from a group consisting of: X-Phos, XantPhos, RuPhos, BrettPhos, SPhos, DavePhos, JohnPhos and tBuXPhos.

In other embodiments of the present invention, the metal catalyst is a palladium catalyst, such as palladium chloride, palladium acetate, tris(dibenzylideneacetone)dipalladium, bis(dibenzylideneacetone)palladium, tetrakis(triphenylphosphine)palladium, dichloro [1,1'-Bis(diphenylphosphino) -ferrocene] palladium, dichlorobis(tricyclohexylphosphine)palladium or dichlorobis(triphenylphosphine)palladium, and preferably, tris-(dibenzylideneacetone)dipalladium, palladium chloride or palladium acetate.

Generally, the reaction can be promoted in alkaline environment (such as potassium acetate, sodium carbonate, potassium carbonate, cesium carbonate, cesium fluoride, sodium hydroxide, potassium hydroxide, potassium phosphate or sodium phosphate, or the like). The solvent is selected from a group consisting of aromatic hydrocarbons, ethers, alcohols, ethers, nitriles, amides and the like, preferably toluene, chlorobenzene, tetrahydrofuran (THF), N,N-dimethylformamide (DMF), dimethyl sulfoxide (DMSO), dioxane, isopropanol, ethanol or acetonitrile; more preferably N,N-dimethylformamide (DMF) and dioxane. The reaction temperature is preferably 60-110 °C, and more preferably 90-110 °C.

When being not 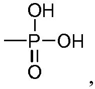 the protective group R can be optionally removed (where R is as defined above, excluding hydrogen), to obtain the compound of the formula below:

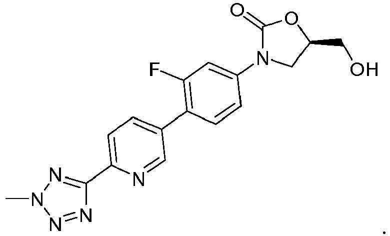

The compound of the formula above can be further phosphorylated to obtain tedizolid phosphate of the formula below:

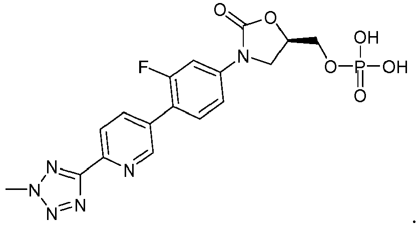

The invention also provides a preparation method of a novel tedizolid intermediate of the formula below: 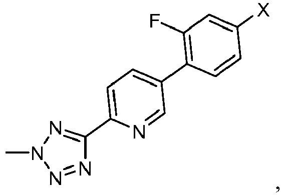
wherein X is chlorine, bromine, iodine, or sulfonyloxy such as trifluoromethane sulfonyloxy, methanesulfonyloxy, benzenesulfonyloxy, or benzenesulfonyloxy substituted by one or more substituents, and the substituents are selected from the group consisting of halogen, C₁-C₆ alkyl and C₁-C₆ alkoxy; preferably, X is chlorine, bromine or iodine; and more preferably, X is bromine or iodine;
wherein the method includes reacting the compound of the formula below 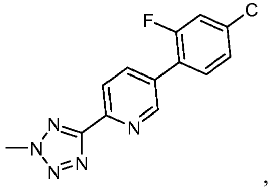
wherein C is hydroxyl group or amino group;
1) with sulfochloride compounds (such as trifluoroformic anhydride, methanesulfonyl chloride or benzenesulfonyl chloride, or the like), when C is hydroxyl, to obtain the compound of the formula below: 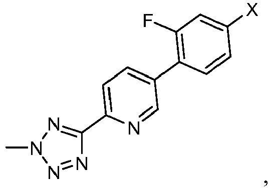
   wherein X is sulfonyloxy such as trifluoromethane sulfonyloxy, methanesulfonyloxy, benzenesulfonyloxy, or benzenesulfonyloxy substituted by one or more substituents, and the substituents are selected from a group consisting of halogen, C₁-C₆ alkyl and C₁-C₆ alkoxy;
   in this circumstance, addition of alkaline substances, such as potassium carbonate, sodium carbonate, cesium carbonate, cesium fluoride, potassium acetate, sodium hydroxide, potassium hydroxide, potassium phosphate, sodium phosphate, or the like, can promote the reaction.
2) to obtain diazonium compounds in the presence of sodium nitrite, when C is amino group, and the diazonium compounds are then substituted by halogen ions to form the compound with of a structure of the formula below: 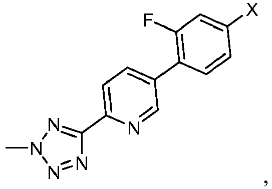 wherein X is selected from a group consisting of chlorine, bromine and iodine;
   in this circumstance, under acidic conditions such as in the presence of acetic acid, methanesulfonic acid, hydrochloric acid, sulfuric acid or camphosulfonic acid, it is reacted with sodium nitrite to form diazonium. Diazonium is then substituted by halogen ions to obtain the compound of the formula above. Donors of the halogen ions can be chlorine, bromine or iodine. Taking iodine as an example, the donors may include iodine element or Iodide salt such as sodium iodide, potassium iodide, or the like.

The invention also provides a method for preparing a novel tedizolid intermediate, *i.e.* the compound of the formula below: 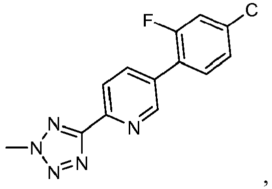 wherein the method includes reacting the compound of the formula below 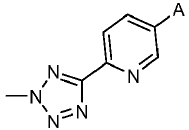 with the compound of the formula below 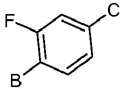 by catalysis of palladium catalyst by a coupled reaction,
wherein C is a hydroxyl or amino group; one of A and B is halogen such as chlorine, bromine, or iodine, or sulfonyloxy such as trifluoromethane sulfonyloxy, methanesulfonyloxy, benzenesulfonyloxy, or benzenesulfonyloxy substituted by one or more substituents, and the substituents are selected from a group consisting of halogen, C₁-C₆ alkyl and C₁-C₆ alkoxy; preferably one of A and B is chlorine, bromine or iodine; and more preferably one of A and B is bromine or iodine, and the other one is BF₃ or BR₂R₃, wherein R₂ and R₃ are independently selected from a group consisting of OH and C₁-C₆ monohydric alcohol and C₁-C₆ diol or C₁-C₆ monohydric alcohol and C₁-C₆ diol substituted by halogen, and wherein R₂ and R₃ can form a ring.

In one embodiment, preferably A is halogen such as chlorine, bromine, or iodine, or sulfonyloxy such as trifluoromethane sulfonyloxy, methanesulfonyloxy, benzenesulfonyloxy, or benzenesulfonyloxy substituted by one or more substituents, and the substituents are selected from a group consisting of halogen, C₁-C₆ alkyl and C₁-C₆ alkoxy; preferably A is chlorine, bromine or iodine; and more preferably A is bromine or iodine, B is BF₃ or BR₂R₃, wherein R₂ and R₃ can be independently selected from a group consisting of OH and C₁-C₆ monohydric alcohol and C₁-C₆ diol or C₁-C₆ monohydric alcohol and C₁-C₆ diol substituted by halogen, and wherein R₂ and R₃ can form a ring; preferably, BR₂R₃ is B(OH)₂ or

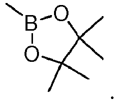

In another embodiment, preferably B is halogen such as chlorine, bromine, or iodine, or sulfonyloxy such as trifluoromethane sulfonyloxy, methanesulfonyloxy, benzenesulfonyloxy, or benzenesulfonyloxy substituted by one or more substituents, and the substituents are selected from a group consisting of halogen, C₁-C₆ alkyl and C₁-C₆ alkoxy; preferably B is chlorine, bromine or iodine; and more preferably B is bromine or iodine, A is BF₃ or BR₂R₃, wherein R₂ and R₃ can be independently selected from a group consisting of OH and C₁-C₆ monohydric alcohol and C₁-C₆ diol or C₁-C₆ monohydric alcohol and C₁-C₆ diol substituted by halogen, and wherein R₂ and R₃ can form a ring; preferably, BR₂R₃ is B(OH)₂ or

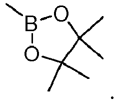

In one embodiment, C is a hydroxyl or amino group; preferably B is bromine or iodine, and A is BF₃, B(OH)₂ or

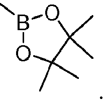

In an embodiment, the catalyst for reaction is palladium catalyst. The palladium catalyst is palladium chloride, palladium acetate, bis(dibenzylideneacetone) palladium, tetrakis(triphenylphosphine) palladium, dichloro[1,1'-bis-(diphenylphosphino)ferrocene] palladium, dichlorobis(tricyclohexylphosphine) palladium or dichlorobis(triphenylphosphine) palladium, or the like.

The reaction can be promoted in the presence of alkaline substances, such as potassium carbonate, sodium carbonate, cesium carbonate, cesium fluoride, potassium acetate, sodium hydroxide, potassium hydroxide, potassium phosphate or sodium phosphate. The solvent can be a combination of one or more selected from the group consisting of water, toluene, tetrahydrofuran (THF), N,N-dimethylformamide (DMF), dimethyl sulfoxide (DMSO), 1,4-dioxane, isopropanol, ethanol, acetonitrile, or the like, and preferably toluene, water and dioxane, or isopropanol. The reaction temperature is preferably 50-120 °C, and more preferably 70-100 °C.

In another embodiment, C is a hydroxyl or amino group; and preferably A is bromine or iodine, and B is BF₃, B(OH)₂ or

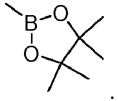

In one embodiment, the reaction is carried out preferably in the presence of palladium catalyst. The solvent is preferably water and dioxane, and the reaction temperature is about 60-80 °C.

Then the tedizolid intermediate compound below is prepared:

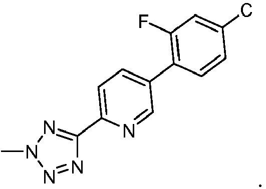

The invention also provides a novel tedizolid intermediate, *i.e.* the compound of the chemical formula below: 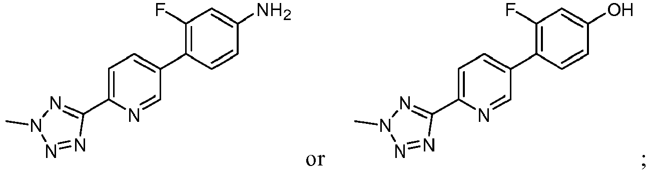 or the compound of the chemical formula below: 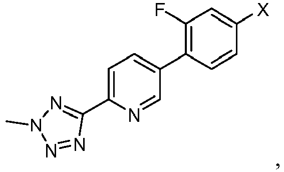 wherein X is chlorine, bromine, iodine, or sulfonyloxy such as trifluoromethane sulfonyloxy, methanesulfonyloxy, benzenesulfonyloxy, or benzenesulfonyloxy substituted by one or more substituents selected from the group consisting of halogen, C₁-C₆ alkyl and C₁-C₆ alkoxy); preferably X is bromine or iodine. The compound is preferably as follows:

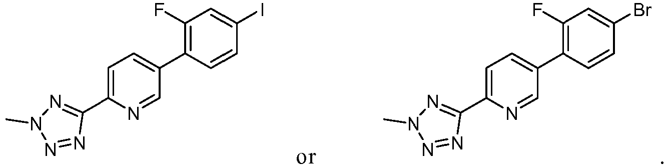

The invention achieves the following technical effects: by comparison with the prior art, the present invention provides a novel method for preparing tedizolid, having the advantages of material availability, low cost, a high yield for each step, simple process, easy operation and environmental friendliness, economical efficiency and being conducive to industrial production. The preparation method of the present invention involves the use of a key intermediate 5-(4-substituent group-2-fluorophenyl)-2-(2-methyl-2H-tetrazol-5-yl)pyridine which allows the preparation scheme of tedizolid being carried out.

### DETAILED DESCRIPTION OF THE INVENTION

### Examples

In order to make the technical problems solved by the invention, the technical solutions and the beneficial effects more clearly, the invention will be further illustrated in combination with specific examples. The specific examples given are preferred examples of the invention.

### Experiments and data analysis

Reagents are purchased from commercial sources and they are directly used without treatment. ¹H-NMR spectra were measured on a Bruker AVANCE 400 spectrometer operating at 400 MHz. MS data were recorded by Agilent HPLC 1260 Infinity and 6120 Duadrupole LC/MS.

### Example 1: Preparation of 2-(2-methyl-2H-tetrazol-5-yl)-5-(4,4,5,5-tetramethyl- 1,3,2-dioxaborolan-2-yl)pyridine

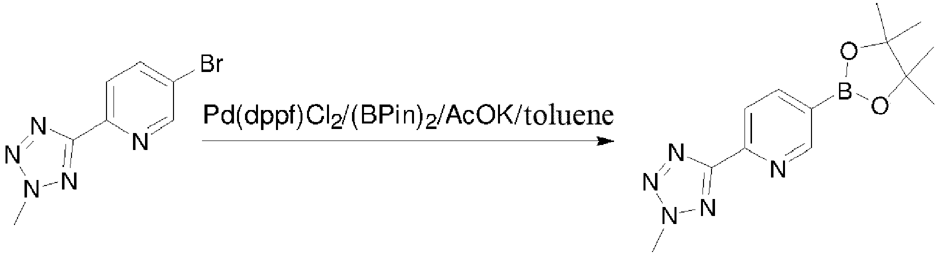

5-bromo-2-(2-methyl-2H-tetrazol-5-yl)pyridine (20.0 g, 1 eq), bis(pinacolato)- diboron (42.3 g, 2.0 eq), potassium acetate (24.5 g, 3.0 eq) and toluene (400 mL) were added into a three-necked flask equipped with agitator and thermometer. After purging with N₂, Pd(dppf)Cl₂ (0.6 g, 3%w/w) was added, followed by purging with N₂ again, and the mixture was reacted under stirring at 80-85 °C for 12 h. The completion of the reaction was monitored by HPLC. The reaction liquid was cooled to 40-50 °C, suction filtered at this temperature. 5 g activated carbon was added to the filtrate. The mixture was then heated to 70-80 °C under stirring for 1-2 h for decoloration, cooled to 40-50°C, and suction filtered at this temperature. The obtained filtrate was distillated under reduced pressure to 40-60 mL, cooled to 10-15 °C to separate out white solid. The white solid was filtered and the filter cake was oven dried at 50 °C to obtain 19.5 g white solid product with a yield of 81.5% and an HPLC purity of 98.5%. LCMS[M+H]=288.1, NMR(CDCl₃, 400MHz): 9.15(t, 1H), 8. 22(m, 2H), 4.44(s, 3H), 1.25(s, 12H).

### Example 2: Preparation of 3-fluoro-4-(6-(2-methyl-2H-tetrazol-5-yl)pyridyl-3-yl) -aniline

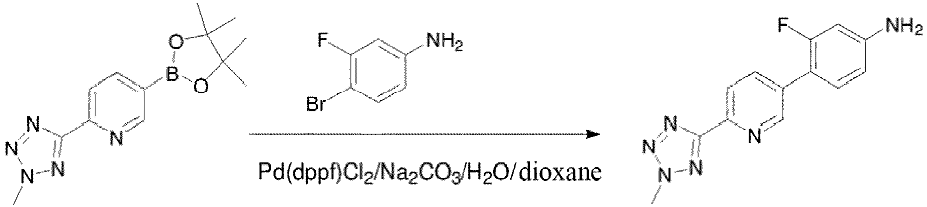

2-(2-methyl-2H-tetrazol-5-yl)-5-(4,4,5,5-tetramethyl-1,3,2-dioxaborolan-2-yl)pyr-idine (50 g, 1 eq), 4-bromo-3-fluoroaniline (36.4 g, 1.1 eq), Na₂CO₃ (36.9 g, 2.0 eq), water (300 mL) and dioxane (1000mL) were added into a three-necked flask equipped with agitator and thermometer. After purging with N₂, Pd(dppf)Cl₂ (1.5 g, 3%w/w) was added, followed by purging with N₂ again, and the mixture was reacted under stirring at 70-80 °C for 12 h. The completion of the reaction was monitored by HPLC. The mixture obtained was distilled under reduced pressure to remove most of the dioxane, added with 500 mL water, stirred at room temperature for 2-3 h; and then filtered. The filter cake was pulped with ethanol (100 mL), and then filtered. The obtained filter cake was oven dried at 50 °C to obtain 42.3 g offwhite solid products with a yield of 90% and an HPLC purity of 99.1%. LCMS[M+H]=271.0, NMR(DMSO-d6, 400MHz): 9.01(t, 1H), 8.54(s, 2H), 8.18 (m, 2H), 7.75(t, 1H), 7.59 (d, 1H), 7.29 (d, 1H), 4.41(s, 3H).

### Example 3: Preparation of 5-(2-fluoro-4-iodophenyl)-2-(2-methyl-2H-tetrazol- 5-yl)pyridine

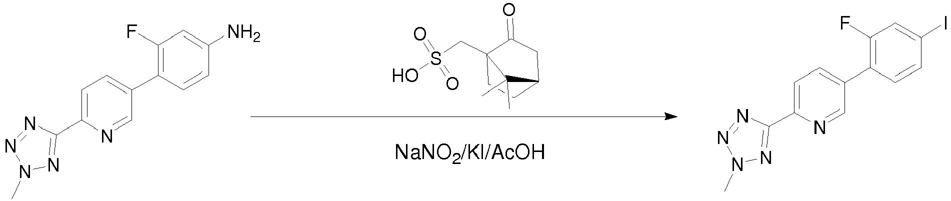

3-fluoro-4-(6-(2-methyl-2H-tetrazol-5-yl) pyridin-3-yl)aniline (30.0 g, 1 eq) and acetic acid (600 mL) were added into a three-necked flask equipped with agitator and thermometer, and dissolved under stirring at room temperature. Then, camphorsulfonic acid (30.9 g, 1.2 eq), potassium iodide (36.9 g, 2.0 eq) and sodium nitrite (9.2 g, 1.2 eq) were successively added. The mixture obtained was stirred for 16 h at room temperature. The completion of the reaction was monitored by HPLC. The mixture obtained was distilled under reduced pressure to remove most of the acetic acid, then added with 300 mL water and 500 mL dichloromethane, stirred and separated. The dichloromethane layer was washed with water, and then distilled under reduced pressure to remove solvent. 20.8 g brown solid product was obtained with a yield of 49% and an HPLC purity of 96.7%. LCMS[M+H]=381.9, NMR(DMSO-d6, 400MHz): 8.85(s, 1H), 8.28 (d, 1H), 8.01 (d, 1H), 7.65(t, 1H), 7.56 (dd, 1H), 7.19 (d, 1H), 4.42(s, 3H).

### Example 4: Preparation of (R)-3-(3-fluoro-4-(6-(2-methyl-2H-tetrazol-5-yl)pyridyl-3-yl)phenyl)-5-(hydroxymethyl)oxazolidin-2-one

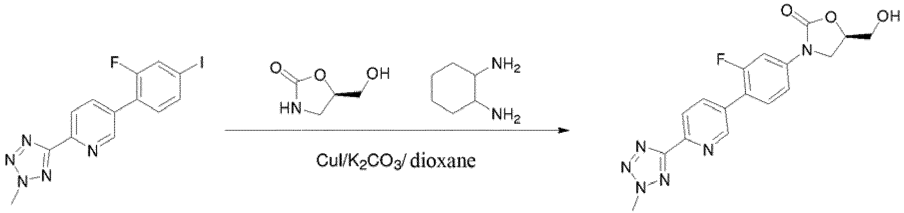

5-(2-fluoro-4-iodophenyl)-2-(2-methyl-2H-tetrazol-5-yl)pyridine (50.0 g, 1 eq), (R)-5-(hydroxymethyl)oxazolidin-2-one (23.0 g, 1.5 eq), cyclohexanediamine (1.5 g, 0.1 eq), cuprous iodide (1.3 g, 0.05 eq), potassium carbonate (36.3 g, 2.0 eq) and dioxane (500 mL) were added into a three-necked flask equipped with agitator and thermometer. After purging with N₂, the mixture obtained was reacted under stirring at 100-110 °C for 16 h. The completion of the reaction was monitored by HPLC. The mixture obtained was distilled under reduced pressure to remove most of the solvent. 1000 mL water was added into the residues, heated to reflux for 1-2 h, then cooled to 70 °C, and suction filtered. The filter cake was pulped with DMF (150 mL) for 2 h, and then filtered. The filter cake was pulped again with water (300 mL) and then filtered. The filter cake was oven dried at 65 °C to obtain offwhite solid products with a yield of 79.2% and an HPLC purity of 97.9%. LCMS[M+H]=371.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

### Example 5: Preparation of 3-fluoro-4-(6-(2-methyl-2H-tetrazol-5-yl)pyridyl -3-yl)phenol

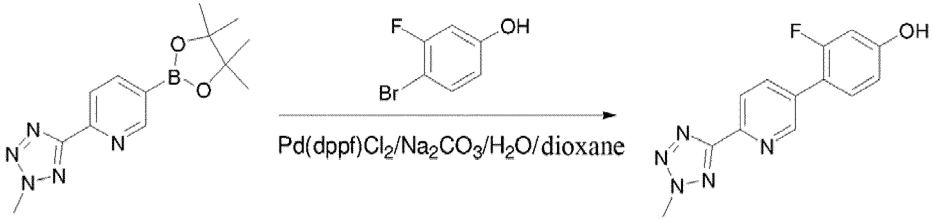

2-(2-methyl-2H-tetrazol-5-yl)-5-(4,4,5,5-tetramethyl-1,3,2-dioxaborolan-2-yl)pyr-idine (25 g, 1 eq), 4-bromo-3-fluorophenol (19.9 g, 1.2 eq), Na₂CO₃ (18.5 g, 2.0 eq), water (120 mL) and dioxane (600 mL) were added into a three-necked flask equipped with agitator and thermometer. After purging with N₂, Pd(dppf)Cl₂ (0.75 g, 3%w/w) was added, followed by purging with N₂ again, and the mixture was reacted under stirring at 70-80 °C for 12 h. The completion of the reaction was monitored by HPLC. The mixture was distilled under reduced pressure to remove most of the dioxane. 500 mL water was added, stirred at room temperature for 2-3 h, and then filtered. The filter cake was pulped with isopropanol (70 mL). The mixture obtained was filtered, and the filter cake was oven dried at 50 °C to obtain 18.2 g white solid product with a yield of 77% and an HPLC purity of 99.5%. LCMS[M+H]=272.0, NMR(DMSO-d6, 400MHz): 10.20(s, 1H), 9.11(s, 1H), 8.54(d, 1H), 8.18 (d, 1H), 7.75(t, 1H), 7.59 (d, 1H), 7.29 (d, 1H), 4.39(s, 3H).

### Example 6: Preparation of 3-fluoro-4-(6-(2-methyl-2H-tetrazol-5-yl)pyridyl- 3-yl)phenyl trifluoromethanesulfonate

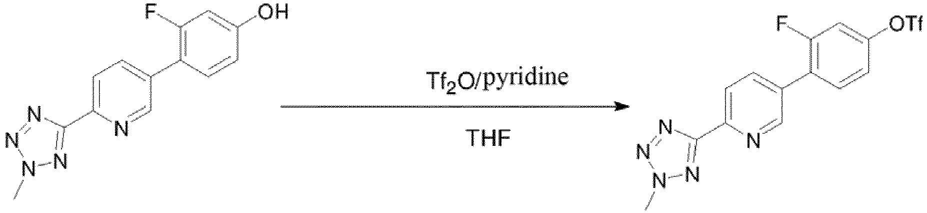

3-fluoro-4-(6-(2-methyl-2H-tetrazol-5-yl)pyridin-3-yl)phenol (15 g, 1 eq), trifluoromethanesulfonic anhydride (23.4 g, 1.5 eq), pyridine (6.6 g, 1.5 eq), and tetrahydrofuran (150 mL) were added into a three-necked flask equipped with agitator and thermometer. After purging with N₂, the mixture obtained was heated to reflux and react for 5 h under stirring. The completion of the reaction was monitored by HPLC. The mixture obtained was distilled under reduced pressure to remove most of the tetrahydrofuran. Then, 100 mL ethyl acetate and 100 mL water were added, and stirred at room temperature. Liquids were separated. The organic phase was dried and distilled to remove solvent. 20.0 g white solid product was obtained with a yield of 90% and an HPLC purity of 96.3%. LCMS[M+H]=403.9, NMR(DMSO-d6, 400MHz): 9.12(s, 1H), 8.44(d, 1H), 8.08 (d, 1H), 7.85(t, 1H), 7.67 (d, 1H), 7.28 (d, 1H), 4.33(s, 3H).

### Example 7: Preparation of (R)-3-(3-fluoro-4-(6-(2-methyl-2H-tetrazol-5-yl)pyridyl-3-yl)phenyl)-5-(hydroxymethyl)oxazolidin-2-one

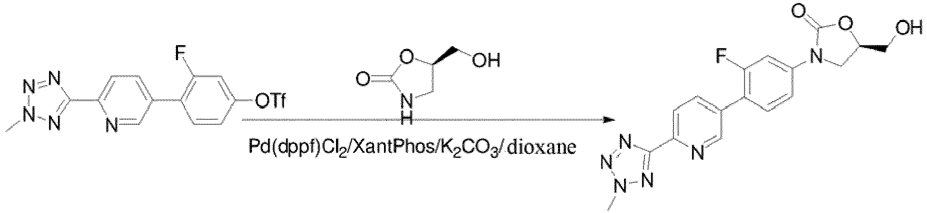

3-fluoro-4-(6-(2-methyl-2H-tetrazol-5-yl)pyridin-3-yl)phenyl trifluoromethanesulfonate (10.0 g, 1 eq), (R)- 5-(hydroxymethyl)oxazolidin -2-one (3.8 g, 1.3 eq), Pd(dppf)Cl₂ (300 mg, 3% w/w), XantPhos (300 mg, 3% w/w), potassium carbonate (6.9 g, 2.0 eq) and dioxane (80 mL) were added into a three-necked flask equipped with agitator and thermometer. After purging with N₂, the mixture obtained was reacted under stirring at 100-110 °C for 16 h. The completion of the reaction was monitored by HPLC. The mixture obtained was distilled under reduced pressure to remove most of the solvent. The residue was added with 100 mL water, heated to reflux for 1-2 h, cooled to 70 °C, and filtered under reduced pressure. the filter cake was pulped with DMF (150 mL) for 2 h. The obtained mixture was filtered, and the filter cake was pulped with water (150 mL) again. The obtained mixture was filtered, and the filter cake was oven dried at 65 °C to obtain 6.1 g offwhite solid product with a yield of 66% and an HPLC purity of 96.9%. LCMS[M+H]=371.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

### Example 8: Preparation of tedizolid phosphate

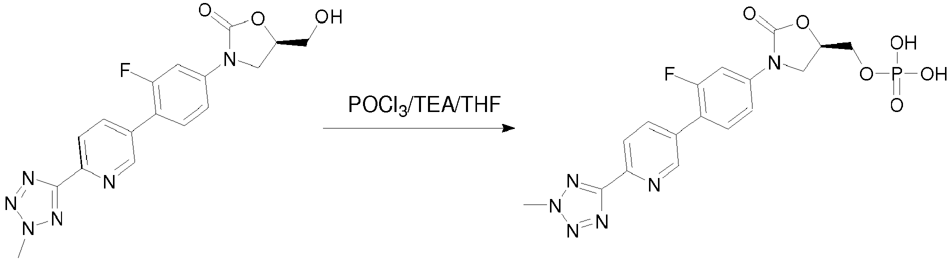

(R)-3-(3-fluoro-4-(6-(2-methyl-2H-tetrazol-5-yl)pyridin-3-yl)phenyl)-5-(hydroxymethyl)-oxazo lidin-2-one (10.0 g, 1 eq) and tetrahydrofuran (200 mL) were added into a three-necked flask equipped with agitator and thermometer. After purging with N₂, the mixture obtained was cooled to 0°C. Triethylamine (8.2 g, 3 eq) was added dropwise. Phosphorus oxychloride was added dropwise at 0-10°C under stirring. After the addition was completed, the reaction mixture was warmed to room temperature slowly and reacted for 20 h. The completion of the reaction was monitored by HPLC. The reaction liquid was added into 100 mL ice water dropwise slowly, stirred overnight, and filtered. The solid product was washed with 50 mL to obtain filter cake. The filter cake was oven dried at 65 °C for 20 h and obtained crude white solid , which was then pulped with 40 mL methanol, and filtered. The filter cake was oven dried at 65 °C to obtain 6.8 g pure product of white solid with a yield of 56% and an HPLC purity of 99.7%. LCMS[M+H]=451.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

### Example 9: Preparation of 3-fluoro-4-(4,4,5,5-tetramethyl-1,3,2-dioxaborolan-2-yl)aniline

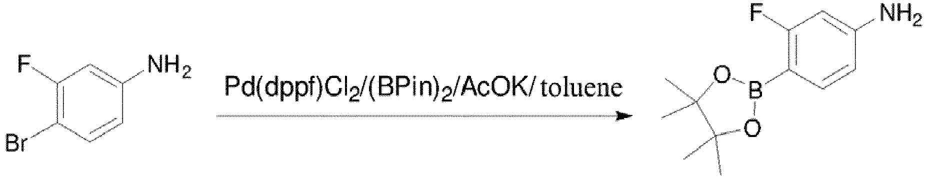

4-bromo-3-fluoroaniline (50.0 g, 1 eq), bis(pinacolato)diboron (100.2 g, 1.5 eq), potassium acetate (77.5 g, 3.0 eq) and toluene (500 mL) were added into a three-necked flask equipped with agitator and thermometer. After purging with N₂, Pd(dppf)Cl₂ (1.5 g, 3%w/w) was added, followed by purging with N₂ again, and the mixture was reacted under stirring at 80-85 °C for 8 h. The completion of the reaction was monitored by HPLC. The reaction liquid was cooled to 40-50 °C, and filtered under reduced pressure at this temperature. 10 g activated carbon was added to the filtrate. The filtrate was heated to 70-80 °C under stirring for 1-2 h for decoloration, then cooled to 40-50°C, and filtered under reduced pressure at this temperature. The filtrate was distilled under reduced pressure to 80-100 mL, cooled to 10-15 °C to separate out white solid, and filtered. The filter cake was oven dried at 50 °C to obtain 54 g white solid product with a yield of 87% and an HPLC purity of 98.9%. LCMS[M+H]=238.1, NMR(DMSO-d6, 400MHz): 7.51(d, 1H), 7.02(s, 1H), 6.87(d, 1H), 6.51(s, 2H), 1.25(s, 12H).

### Example 10: Preparation of 3-fluoro-4-(6-(2-methyl-2H-tetrazol-5-yl)pyridyl- 3-yl)aniline

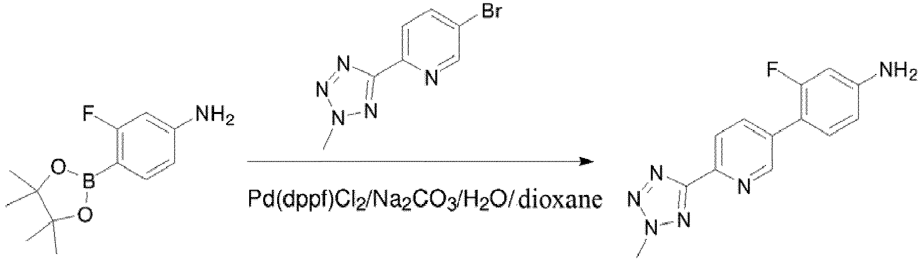

3-fluoro-4-(4,4,5,5-tetramethyl-1,3,2-dioxoborolan-2-yl)aniline (15 g, 1 eq), 5-bromo-2-(2-methyl-2H-tetrazol-5-yl)pyridine (18.2 g, 1.2 eq), Na₂CO₃ (13.4 g, 2.0 eq), water (60 mL) and dioxane (300 mL) were added into a three-necked flask equipped with agitator and thermometer. After purging with N₂, Pd(dppf)Cl₂ (450 mg, 3%w/w) was added, followed by purging with N₂ again. The mixture was reacted under stirring at 70-80 °C for 12 h. The completion of the reaction was monitored by HPLC. The obtained mixture was distilled under reduced pressure to remove most of the dioxane, added with 150 mL water, stirred at room temperature for 2-3 h, and filtered. The filter cake was pulped with ethanol (80 mL). The mixture obtained was filtered, and the filter cake was oven dried at 50 °C to obtain 14.8 g offwhite solid product with a yield of 87% and an HPLC purity of 98.3%. LCMS[M+H]=271.0, NMR(DMSO-d6, 400MHz): 9.01(t, 1H), 8.54(s, 2H), 8.18 (m, 2H), 7.75(t, 1H), 7.59 (d, 1H), 7.29 (d, 1H), 4.41(s, 3H).

### Example 11: Preparation of 3-fluoro-4-(4,4,5,5-tetramethyl-1,3,2-dioxaborolan-2-yl)phenol

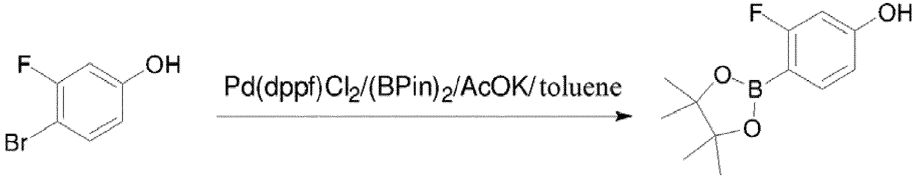

4-Bromo-3-fluorophenol (30.0 g, 1 eq), bis(pinacolato)diboron (59.8 g, 1.5 eq), potassium acetate (46.3 g, 3.0 eq) and toluene (300 mL) were added into a three-necked flask equipped with agitator and thermometer. After purging with N₂, Pd(dppf)Cl₂ (0.9 g, 3%w/w) was added, followed by purging with N₂ again, and the mixture was reacted under stirring at 80-85 °C for 8 h. The completion of the reaction was monitored by HPLC. The reaction liquid was cooled to 40-50 °C, filtered under reduced pressure at this temperature. 10 g activated carbon was added to the filtrate. The filtrate was heated to at 70-80 °C under stirring for 1-2 h for decoloration, then cooled to 40-50°C, and filtered under reduced pressure at this temperature. The filtrate was distilled under reduced pressure to 60-80 mL, cooled to 0-10 °C to separate out white solid, and filtered. The filter cake was oven dried at 50 °C to obtain 29.8 g white solid product with a yield of 80% and an HPLC purity of 99.5%. LCMS[M+H]=239.0, NMR(DMSO-d6, 400MHz): 10.50(s, 1H), 7.65(d, 1H), 7.42(s, 1H), 7.01(d, 1H), 1.26(s, 12H).

### Example 12: Preparation of 3-fluoro-4-(6-(2-methyl-2H-tetrazol-5-yl)pyridyl- 3-yl)phenol

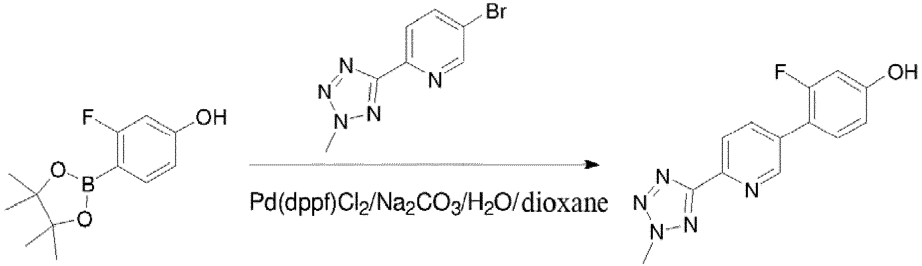

3-fluoro-4-(4,4,5,5-tetramethyl-1,3,2-dioxoboran-2-yl)phenol (18.0 g, 1 eq), 5-bromo-2 -(2-methyl-2H-tetrazol-5-yl)pyridine (21.8 g, 1.2 eq), Na₂CO₃ (16.0 g, 2.0 eq), water (54 mL) and dioxane (270 mL), were added into a three-necked flask equipped with agitator and thermometer. After purging with N₂, Pd(dppf)Cl₂ (540 mg, 3%w/w) was added, followed by purging with N₂ again, and the mixture was reacted under stirring at 70-80 °C for 12 h. The completion of the reaction was monitored by HPLC. The obtained mixture was distilled under reduced pressure to remove most of the dioxane, added with 300 mL water, stirred at room temperature for 2-3 h, and filtered. The filter cake was pulped with isopropanol (90 mL). The obtained mixture was filtered, and the filter cake was oven dried at 50 °C to obtain 16.0 g white solid product with a yield of 78% and an HPLC purity of 98.9%. LCMS[M+H]=272.0, NMR(DMSO-d6, 400MHz): 10.20(s, 1H), 9.11(s, 1H), 8.54(d, 1H), 8.18 (d, 1H), 7.75(t, 1H), 7.59 (d, 1H), 7.29 (d, 1H), 4.39(s, 3H).

### Example 13: Preparation of (R)-2-((benzyloxy)methyl)ethylene oxide

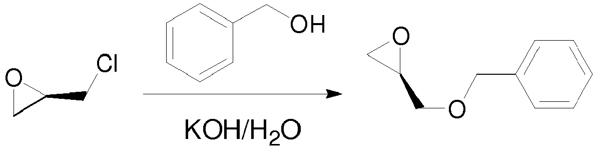

Benzyl alcohol (50 g, 1 eq), potassium hydroxide aqueous solution 300 mL (50% w/w), TBAB (14.9 g, 0.1 eq) and dichloromethane (300 mL) were added into a three-necked flask equipped with agitator and thermometer, and cooled to 0-10 °C. Epoxy chloropropane (64.2 g, 1.5 eq) was added dropwise slowly. After the addition was completed, the mixture obtained was warmed to room temperature and reacted for 16 h. The completion of the reaction was monitored by HPLC. The stir was stopped and the liquids were separated. The aqueous phase was extracted once with 300 mL dichloromethane; and the organic phases were combined and directly used in the next step without purification.

### Example 14: Preparation of (R)-1-amino-3-(benzyloxy)isopropanol

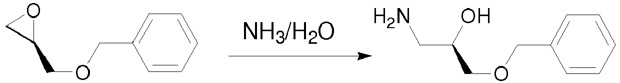

A solution of (R)-2-((benzyloxy)methyl)oxirane in dichloromethane and 100 mL aqueous ammonia were added into a hydrogenated bottle equipped with agitator, heated to 35 °C after sealing, stirred and reacted for 16 h. The completion of the reaction was monitored by HPLC. The stir was stopped, and 300mL water was added, and stirred. The liquids were separated. The organic phase was washed with 0.1M HCl solution (100mL), and dichloromethane was separated and discarded. The aqueous phase was adjusted with NaOH to pH 9-10, extracted with (300mL) dichloromethane and concentrated to obtain 55.0 g colorless liquid product, with a two-step yield of 66% and an HPLC purity of 98.4%. LCMS[M+H]=182.0, NMR(DMSO-d6, 400MHz): 7.42-7.25(m, 5H), 5.11(s, 2H), 4.54(s, 2H), 3.68 (m, 1H), 3.50-3.34(m, 2H), 3.12-3.06 (br, 1H), 3.00-2.89 (m, 2H).

### Example 15: Preparation of (R)-5-((benzyloxy)methyl)oxazolidin-2-one

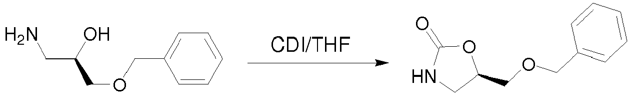

(R)-1-amino-3-(benzyloxy)isopropanol (20 g, 1 eq) and tetrahydrofuran (200 mL) were added into a three-necked flask equipped with agitator, heated to 35 °C, added with CDI (26.8 g, 1.5 eq). The obtained mixture was stirred and reacted for 16 h while keeping the temperature. The completion of the reaction was monitored by HPLC. The stir was stopped and the tetrahydrofuran-containing solution was concentrated. 200 mL ethyl acetate and 100 mL 1M hydrochloric acid were added, and stirred. The liquids were separated. The organic phase was washed with water and the ethyl acetate-containing phase was concentrated to obtain a colorless liquid product 21.0 g with yield of 92% and HPLC purity of 99.0%. LCMS[M+H]=208.0, NMR(DMSO-d6, 400MHz): 8.05(s, 1H),7.44-7.22(m, 5H), 4.56(s, 2H), 4.28-4.20 (m, 1H), 3.77-3.69(m, 1H), 3.42-3.29 (m, 2H), 3.11-3.06 (m, 1H).

### Example 16: Preparation of (R)-5-(hydroxymethyl)oxazolidin-2-one

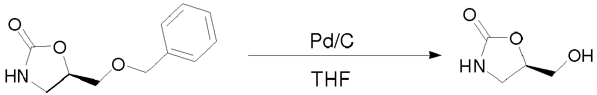

(R)-5-((benzyloxy)methyl)oxazolidin-2-one (15 g, 1 eq), tetrahydrofuran (150 mL) and Pd/C (1.5 g, 10% w/w) were added into a three-necked flask equipped with agitator. After purging with H₂, the mixture obtained was heated to 45 °C, stirred and reacted for 3 h by keeping this temperature. The completion of the reaction was monitored by HPLC. The mixture obtained was filtered without stir to remove Pd/C. The tetrahydrofuran-containing phase was concentrated to obtain 8.4 g colorless oil product with a yield of 99% and an HPLC purity of 98.6%. LCMS[M+H]=117.9, NMR(DMSO-d6, 400MHz): 7.92(s, 1H), 4.68-4.60 (m, 1H), 3.97-3.90(m, 1H), 3.70 (br, 1H), 3.62-3.55 (m, 2H), 3.11-3.06 (m, 1H).

### Example 17: Preparation of (R)-3-(3-fluoro-4-(6-(2-methyl-2H-tetrazol-5-yl)pyridyl-3-yl)phenyl)-5-(hydroxymethyl)oxazolidin-2-one

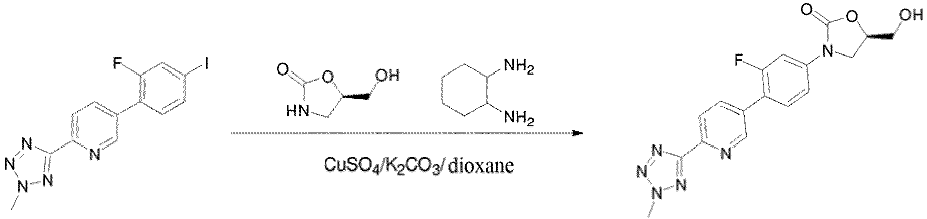

5-(2-fluoro-4-iodophenyl)-2-(2-methyl-2H-tetrazol-5-yl)pyridine (50.0 g, 1 eq), (R)-5-(hydroxymethyl) oxazolidin-2-one (23.0 g, 1.5 eq), cyclohexanediamine (1.5 g, 0.1 eq), copper sulfate (1.05 g, 0.05 eq), potassium carbonate (36.3 g, 2.0 eq) and dioxane (500 mL) were added into a three-necked flask equipped with agitator and thermometer. The mixture obtained was stirred and reacted at 100-110 °C for 16 h. The completion of the reaction was monitored by HPLC. The mixture obtained was distilled under reduced pressure to remove most of the solvent. 1000 mL water was added into the residue, heated to reflux for 1-2 h, then cooled to 70 °C, and filtered under reduced pressure. The filter cake was pulped with DMF (150 mL) for 2 h. The mixture obtained was filtered, and the filter cake was pulped with water (300 mL) again. The mixture obtained was filtered; the filter cake was oven dried at 65 °C to obtain 36.3 g offwhite solid product with a yield of 74.7% and an HPLC purity of 98.3%. LCMS[M+H]=371.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

### Example 18: Preparation of (R)-3-(3-fluoro-4-(6-(2-methyl-2H-tetrazol-5-yl)pyridyl-3-yl)phenyl)-5-(hydroxymethyl)oxazolidin-2-one

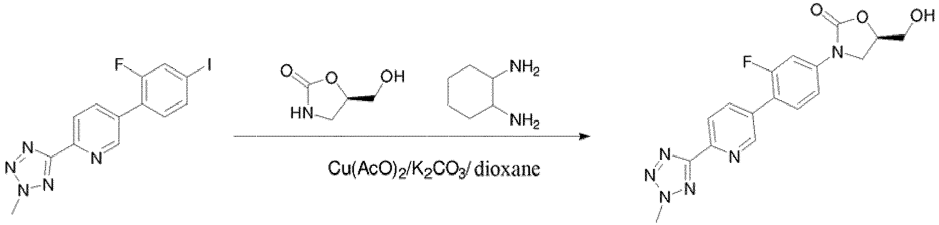

5-(2-fluoro-4-iodophenyl)-2-(2-methyl-2H-tetrazol-5-yl)pyridine (50.0 g, 1 eq), (R)-5-(hydroxymethyl) oxazolidin-2-one (23.0 g, 1.5 eq), cyclohexanediamine (1.5 g, 0.1 eq), Cu(OAc)₂ (1.19 g, 0.05 eq), potassium carbonate (36.3 g, 2.0 eq) and dioxane (500 mL) were added into a three-necked flask equipped with agitator and thermometer. After purging with N₂, the mixture obtained was stirred and reacted at 100-110 °C for 16 h. The completion of the reaction was monitored by HPLC. The mixture obtained was distilled under reduced pressure to remove most of the solvent. 1000 mL water was added into the residue, heated to reflux for 1-2 h, cooled to 70 °C, and filtered under reduced pressure. The filter cake was pulped with DMF (150 mL) for 2 h. The mixture obtained was filtered; and the filter cake was pulped with water (300 mL) again. The mixture obtained was filtered; and the filter cake was oven dried at 65 °C to obtain 39.9 g offwhite solid products with a yield of 82.1% and an HPLC purity of 97.8%. LCMS[M+H]=371.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

### Example 19: Preparation of (R)-3-(3-fluoro-4-(6-(2-methyl-2H-tetrazol-5-yl)pyridyl-3-yl)phenyl)-5-(hydroxymethyl)oxazolidin-2-one

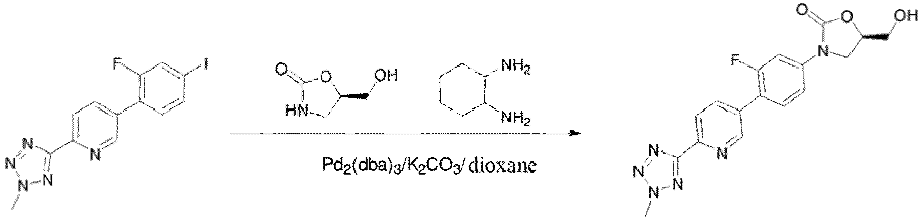

5-(2-fluoro-4-iodophenyl)-2-(2-methyl-2H-tetrazol-5-yl)pyridine (50.0 g, 1 eq), (R)-5-(hydroxymethyl) oxazolidin-2-one (23.0 g, 1.5 eq), cyclohexanediamine (1.5 g, 0.1 eq), tris(dibenzylideneacetone)dipalladium (1.5 g, 3% w/w), potassium carbonate (36.3 g, 2.0 eq) and dioxane (500 mL) were added into a three-necked flask equipped with agitator and thermometer. The mixture obtained was stirred and reacted at 100-110 °C for 16 h. The completion of the reaction was monitored by HPLC. The mixture obtained was distilled under reduced pressure to remove most of the solvent. 1000 mL water was added into the residue, heated to reflux for 1-2 h, cooled to 70 °C, and filtered under reduced pressure. The filter cake was pulped with DMF (150 mL) for 2 h. The mixture obtained was filtered; and the filter cake was pulped with water (300 mL) again. The mixture obtained was filtered; and the filter cake was oven dried at 65 °C to obtain 36.7 g offwhite solid product with a yield of 75.5% and an HPLC purity of 98.0%. LCMS[M+H]=371.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

### Example 20: Preparation of (R)-3-(3-fluoro-4-(6-(2-methyl-2H-tetrazol-5-yl)pyridyl-3-yl)phenyl)-5-(hydroxymethyl)oxazolidin-2-one

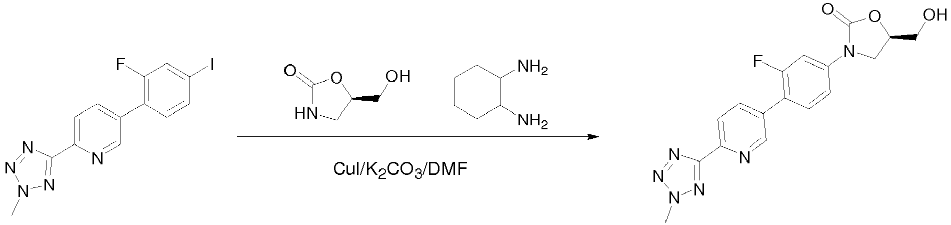

5-(2-fluoro-4-iodophenyl)-2-(2-methyl-2H-tetrazol-5-yl)pyridine (50.0 g, 1 eq), (R)-5-(hydroxymethyl) oxazolidin-2-one (23.0 g, 1.5 eq), cyclohexanediamine (1.5 g, 0.1 eq), cuprous iodide (1.3 g, 0.05 eq), potassium carbonate (36.3 g, 2.0 eq) and DMF (500 mL) were added into a three-necked flask equipped with agitator and thermometer. After purging with N₂, the mixture obtained was stirred and reacted at 100-110 °C for 16 h. The completion of the reaction was monitored by HPLC. The mixture obtained was distilled under reduced pressure to remove most of solvent. 1000 mL water was added into the residue, heated to reflux for 1-2 h, cooled to 70 °C, and filtered under reduced pressure. The filter cake was pulped with DMF (150 mL) for 2 h. The mixture obtained was filtered; and the filter cake was pulped with water (300 mL) again. The mixture obtained was filtered; and the filter cake was oven dried at 65 °C to obtain 36.9 g offwhite solid product with a yield of 76.0% and an HPLC purity of 99.5%. LCMS[M+H]=371.1, NMR(DMSO-d6, 400MHz): 8.95(s, 1H), 8.24 (d, 1H), 8.10 (d, 1H), 7.78(t, 1H), 7.45 (dd, 1H), 7.10 (d, 1H), 4.62(m, 1H), 4.42(s, 3H), 3.84(m, 1H), 3.42-3.35(m, 2H), 3.01 (m, 1H).

## Claims

1. A preparation method for tedizolid compound of the formula below 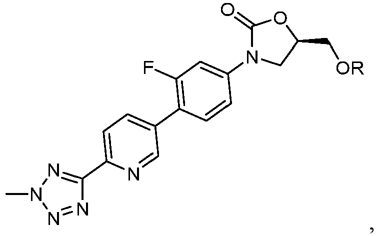
wherein R is selected from the group consisting of hydrogen , 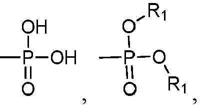 benzyl or benzyl substituted by a substituent which is selected from the group consisting of halogen, nitro, C₁-C₆ alkyl and C₁-C₆ alkoxy, and R₁ is C₁-C₆ alkyl or C₁-C₆ alkyl substituted by halogen;
wherein the preparation method comprises reacting the compound having a structure of the formula below 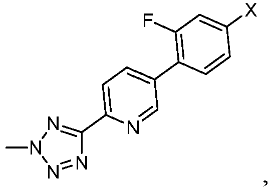
wherein X is chlorine, bromine, iodine, or sulfonyloxy such as trifluoromethane sulfonyloxy, methanesulfonyloxy, benzenesulfonyloxy, or benzenesulfonyloxy substituted by one or more substituent(s) which is selected from the group consisting of halogen, C₁-C₆ alkyl and C₁-C₆ alkoxy;
with the compound having a structure of the formula below 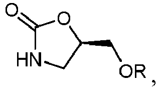 under the catalysis of metal catalyst to produce the tedizolid compound by a coupled reaction, and wherein substituent R is as defined above.

2. The preparation method according to claim 1, wherein the metal catalyst is a copper catalyst or a palladium catalyst; wherein the copper catalyst is preferably selected from the group consisting of CuI, CuBr, CuCl, CuO, Cu₂O, CuSO₄, Cu(OAc)₂, Cu(OTf)₂ or Cu powder, and more preferably CuI or Cu(OAc)₂; and the palladium catalyst is preferably selected from the group consisting of palladium chloride, palladium acetate, bis(dibenzylideneacetone) palladium, tetrakis(triphenylphosphine) palladium, tris(dibenzylideneacetone) dipalladium, dichloro[1,1'-bis(diphenylphosphino)ferrocene] palladium, dichlorobis(tricyclohexylphosphine) palladium or dichlorobis(triphenylphosphine) palladium, and more preferably, tris(dibenzylideneacetone) dipalladium, palladium chloride or palladium acetate.

3. The preparation method according to claim 1 or 2, wherein the coupled reaction is performed in the presence of an alkaline substance as a promoter; and the alkaline substance is preferably selected from the group consisting of potassium carbonate, sodium carbonate, cesium carbonate, cesium fluoride, potassium acetate, sodium hydroxide, potassium hydroxide, potassium phosphate or sodium phosphate.

4. The preparation method according to any one of claims 1-3, wherein the reaction solvent for the coupled reaction is toluene, chlorobenzene, tetrahydrofuran, N,N-dimethylformamide, dimethyl sulfoxide, dioxane, isopropanol, ethanol or acetonitrile, and preferably N,N-dimethylformamide or dioxane; and the reaction temperature is 60-110 °C, and preferably 90-110 °C.

5. The preparation method according to any one of claims 1-4, wherein when the metal catalyst is a copper catalyst, the copper catalyst acts together with ligands which are selected from the group consisting of a diamine ligand, a diketone ligand, a phenanthroline ligand, an amino acid ligand, and a Phos ligand, and preferably the diamine ligand is selected from the group consisting of: 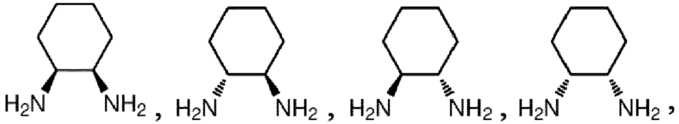 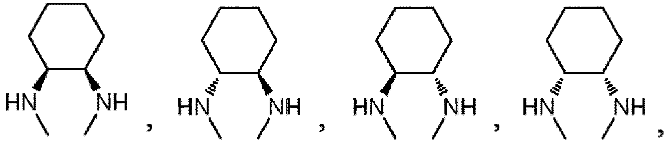 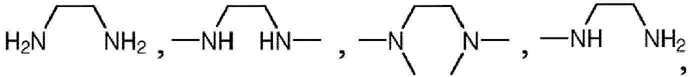 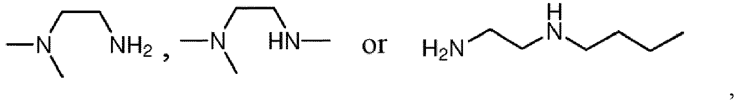 the diketone ligand is selected from the group consisting of: 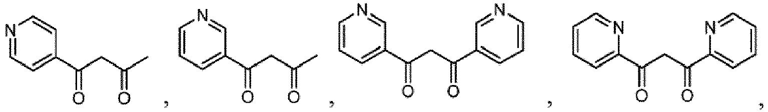 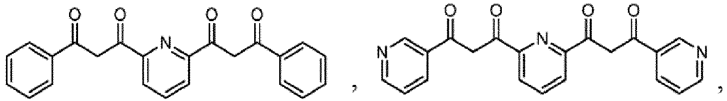 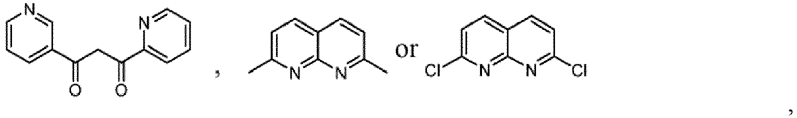 the phenanthroline ligand is selected from the group consisting of: 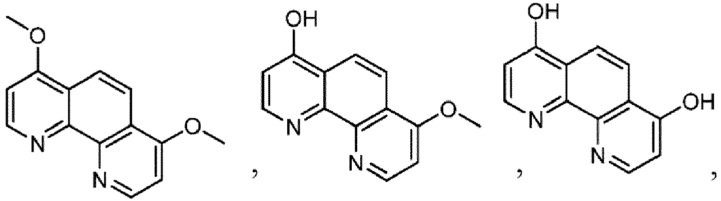 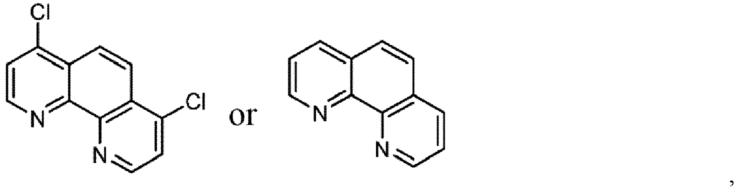 the amino acid ligand is selected from the group consisting of: 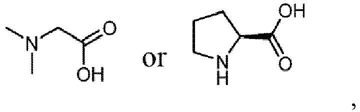 and the Phos ligand is selected from the group consisting of X-Phos, XantPhos, RuPhos, BrettPhos, SPhos, DavePhos, JohnPhos or tBuXPhos.

6. A preparation method for the compound of the formula below 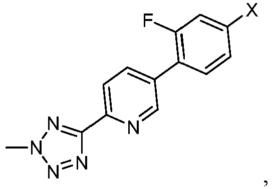
wherein X is chlorine, bromine, iodine, or sulfonyloxy such as trifluoromethane sulfonyloxy, methanesulfonyloxy, benzenesulfonyloxy, or benzenesulfonyloxy substituted by one or more substituent(s) which is selected from the group consisting of halogen, C₁-C₆ alkyl and C₁-C₆ alkoxy;
wherein the preparation method comprises reacting the compound having a structure of the formula below 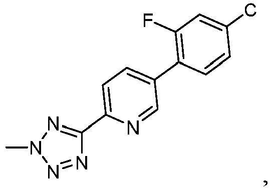
wherein, C is hydroxyl or amino;
1) with sulfonyl chloride compounds, when C is hydroxyl, to produce the compound having a structure of the formula below 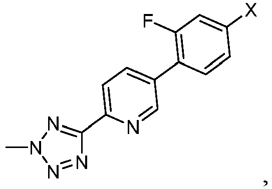 wherein X is sulfonyloxy such as trifluoromethane sulfonyloxy, methanesulfonyloxy, benzenesulfonyloxy, or benzenesulfonyloxy substituted by one or more substituent(s), and wherein the substituent is selected from the group consisting of halogen, C₁-C₆ alkyl and C₁-C₆ alkoxy; or
2) when C is amino, to produce diazonium salt compounds in the presence of sodium nitrite, then being substituted with halogen ion to produce the compound having a structure of the formula below 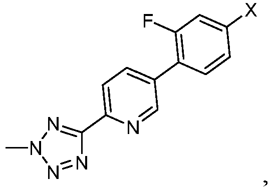 wherein X is chlorine, bromine, or iodine.

7. A preparation method for the compound of the formula below, 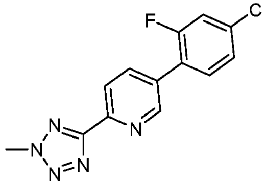 comprising reacting the compound having a structure of the formula below 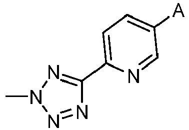 with the compound having a structure of the formula below 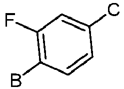 under the catalysis of palladium catalysts by a coupled reaction;
wherein C is hydroxyl or amino; one of A and B is halogen such as chlorine, bromine, or iodine, or sulfonyloxy such as trifluoromethane sulfonyloxy, methanesulfonyloxy, benzenesulfonyloxy, or benzenesulfonyloxy which is substituted by one or more substituents, and the substituent is selected from the group consisting of halogen, C₁-C₆ alkyl and C₁-C₆ alkoxy; preferably one of A and B is chlorine, bromine and iodine; more preferably one of A and B is bromine or iodine, and the other one is BF₃ or BR₂R₃, wherein R₂ and R₃ are independently selected from the group consisting of OH, and C₁-C₆ monohydric alcohol and C₁-C₆ diol or C₁-C₆ monohydric alcohol and C₁-C₆ diol which are substituted by halogen, and wherein R₂ and R₃ can form a ring; preferably BR₂R₃ is B(OH)₂ or 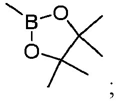 preferably B is halogen such as chlorine, bromine, or iodine, or sulfonyloxy such as trifluoromethane sulfonyloxy, methanesulfonyloxy, benzenesulfonyloxy, or benzenesulfonyloxy which is substituted by one or more substituents, and the substituent is selected from the group consisting of halogen, C₁-C₆ alkyl and C₁-C₆ alkoxy; preferably B is chlorine, bromine or iodine; more preferably B is bromine or iodine,
and A is BF₃ or BR₂R₃, wherein R₂ and R₃ can be independently selected from OH, and C₁-C₆ monohydric alcohol and C₁-C₆ diol or C₁-C₆ monohydric alcohol and C₁-C₆ diol which are substituted by halogen, and wherein R₂ and R₃ can form a ring;
and wherein preferably BR₂R₃ is B(OH)₂ or 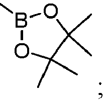 or
preferably A is halogen such as chlorine, bromine, or iodine, or sulfonyloxy such as trifluoromethane sulfonyloxy, methanesulfonyloxy, benzenesulfonyloxy, or benzenesulfonyloxy which is substituted by one or more substituents, and the substituent is selected from the group consisting of halogen, C₁-C₆ alkyl and C₁-C₆ alkoxy; preferably A is chlorine, bromine and iodine; more preferably A is bromine or iodine, and B is BF₃ or BR₂R₃, wherein R₂ and R₃ can be independently selected from OH, and C₁-C₆ monohydric alcohol and C₁-C₆ diol or C₁-C₆ monohydric alcohol and C₁-C₆ diol which are substituted by halogen, and wherein R₂ and R₃ can form a ring;
and wherein preferably BR₂R₃ is B(OH)₂ or

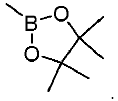

8. The preparation method according to claim 7, wherein the palladium catalyst is palladium chloride, palladium acetate, bis(dibenzylideneacetone) palladium, tetrakis(triphenylphosphine) palladium, dichloro[1,1'-bis(diphenylphosphino)ferrocene] palladium, dichlorobis(tricyclohexylphosphine) palladium or dichlorobis(triphenylphosphine) palladium.

9. The preparation method according to claim 7 or 8, wherein the reaction solvent for the coupled reaction is any one of water, toluene, tetrahydrofuran, N,N-dimethylformamide, dimethyl sulfoxide, dioxane, isopropanol, ethanol, or acetonitrile or any combination thereof, and preferably toluene, water and dioxane, or isopropanol; and the reaction temperature is 50-120 °C, and preferably 70-100 °C.

10. A compound of the chemical formula below: 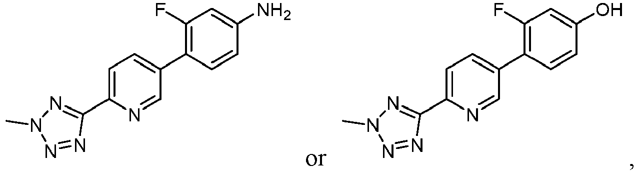 or the chemical formula below: 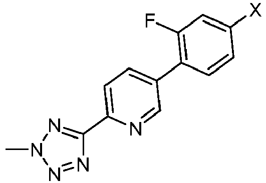 wherein X is chlorine, bromine, iodine, or sulfonyloxy such as trifluoromethane sulfonyloxy, methanesulfonyloxy, benzenesulfonyloxy, or benzenesulfonyloxy substituted by one or more substituent(s) which is selected from the group consisting of halogen, C₁-C₆ alkyl and C₁-C₆ alkoxy; and preferably, X is bromine or iodine, *i.e.* the compound of the formula below:

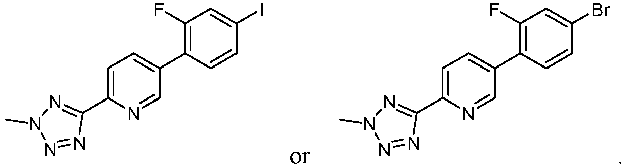

## Patentansprüche

1. Ein Verfahren zur Herstellung einer tedizoiden Verbindung der folgenden Formel 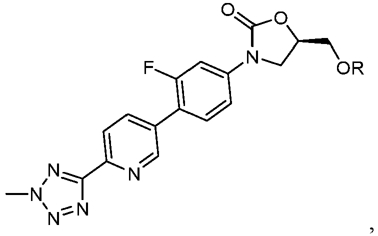 wobei R aus der Gruppe ausgewählt ist bestehend aus Hydrogen , 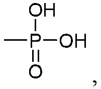 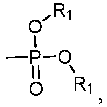 Benzyl oder Benzyl, das mit einem Substituenten substituiert ist, der aus der Gruppe ausgewählt ist bestehend aus Halogen, Nitro, C₁-C₆-Alkyl und C₁-C₆-Alkoxy, und R₁ C₁-C₆-Alkyl ist oder C₁-C₆-Alkyl, das mit Halogen substituiert ist; wobei das Verfahren zur Herstellung umfasst, dass die Verbindung mit einer Struktur der folgenden Formel 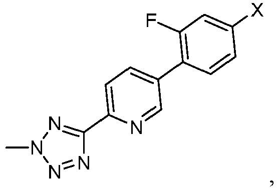 wobei X Chlor, Brom, Jod oder Sulfonyloxy ist, wie Trifluormethansulfonyloxy, Methansulfonyloxy, Benzolsulfonyloxy oder Benzolsulfonyloxy, das mit einem oder mehreren Substituenten substituiert ist, der aus der Gruppe ausgewählt ist bestehend aus Halogen, C₁-C₆-Alkyl und C₁-C₆-Alkoxy;
mit der Verbindung mit einer Struktur der folgenden Formel 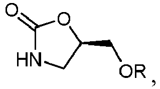 unter der Katalyse eines Metallkatalysators zur Herstellung der tedizoiden Verbindung durch eine gekoppelte Reaktion umgesetzt wird, und wobei der Substituent R wie oben definiert ist.

2. Das Verfahren zur Herstellung nach Anspruch 1, wobei der Metallkatalysator ein Kupferkatalysator oder ein Palladiumkatalysator ist; wobei der Kupferkatalysator bevorzugt aus der Gruppe ausgewählt ist bestehend aus CuI, CuBr, CuCl, CuO, Cu₂O, CuSO₄, Cu(OAc)₂, Cu(OTf)₂ oder Cu-Pulver und noch bevorzugter aus CuI oder Cu(OAc)₂; und der Palladiumkatalysator bevorzugt aus der Gruppe ausgewählt ist bestehend aus Palladiumchlorid, Palladiumacetat, Bis(dibenzylidenaceton)palladium, Tetrakis(triphenylphosphin)palladium, Tris(dibenzylidenaceton)dipalladium, Dichlor[1,1'-bis(diphenylphosphino)ferrocen]palladium, Dichlorbis(tricyclohexylphosphin)palladium oder Dichlorbis(triphenylphosphin)palladium, und noch bevorzugter Tris(dibenzylidenaceton)dipalladium, Palladiumchlorid oder Palladiumacetat.

3. Das Verfahren zur Herstellung nach Anspruch 1 oder 2, wobei die gekoppelte Reaktion in Gegenwart einer alkalischen Substanz als Promotor durchgeführt wird; und die alkalische Substanz bevorzugt aus der Gruppe ausgewählt ist bestehend aus Kaliumcarbonat, Natriumcarbonat, Cäsiumcarbonat, Cäsiumfluorid, Kaliumacetat, Natriumhydroxid, Kaliumhydroxid, Kaliumphosphat oder Natriumphosphat.

4. Das Verfahren zur Herstellung nach einem der Ansprüche 1-3, wobei das Reaktionslösungsmittel für die gekoppelte Reaktion Toluol, Chlorbenzol, Tetrahydrofuran, N,N-Dimethylformamid, Dimethylsulfoxid, Dioxan, Isopropanol, Ethanol oder Acetonitril und bevorzugt N,N-Dimethylformamid oder Dioxan ist; und die Reaktionstemperatur 60-110 °C und bevorzugt 90-110 °C beträgt.

5. Das Verfahren zur Herstellung nach einem der Ansprüche 1-4, wobei, wenn der Metallkatalysator ein Kupferkatalysator ist, der Kupferkatalysator zusammen mit Liganden wirkt, die aus der Gruppe ausgewählt sind bestehend aus einem Diaminliganden, einem Diketonliganden, einem Phenanthrolinliganden, einem Aminosäureliganden und einem Phos-Liganden, und bevorzugt der Diaminligand aus der Gruppe ausgewählt ist bestehend aus: 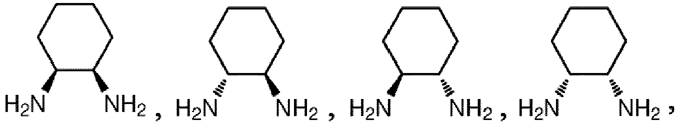 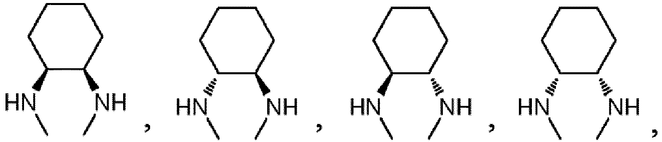 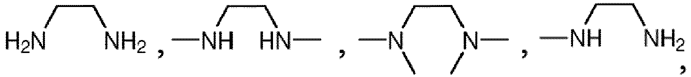 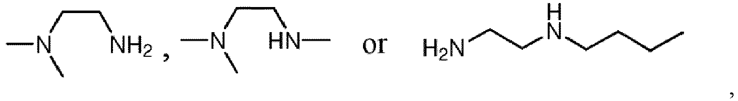 der Diketon-Ligand aus der Gruppe ausgewählt ist bestehend aus: 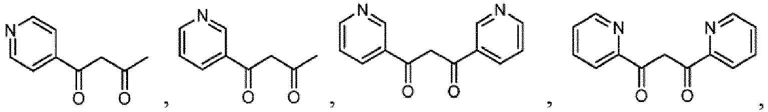 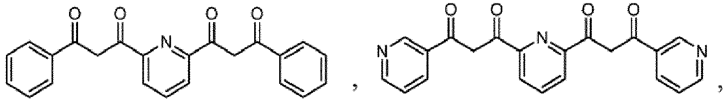 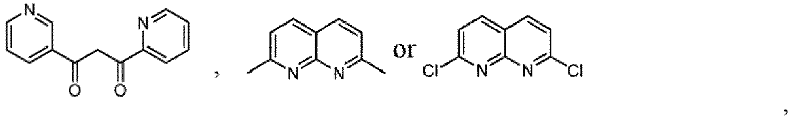 der Phenanthrolin-Ligand aus der Gruppe ausgewählt ist bestehend aus: 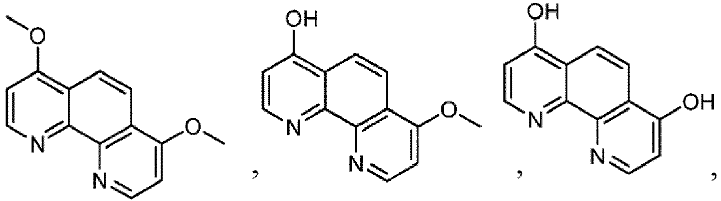 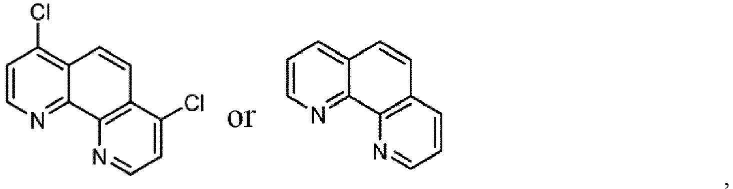 der Aminosäure-Ligand aus der Gruppe ausgewählt ist bestehend aus: 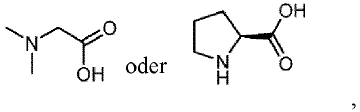 und der Phos-Ligand aus der Gruppe ausgewählt ist bestehend aus X-Phos, XantPhos, RuPhos, BrettPhos, SPhos, DavePhos, JohnPhos oder tBuXPhos.

6. Verfahren zur Herstellung der Verbindung mit der folgenden Formel 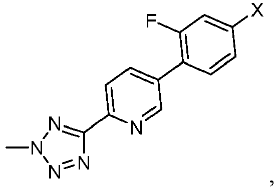
wobei X Chlor, Brom, Jod oder Sulfonyloxy ist, wie Trifluormethansulfonyloxy, Methansulfonyloxy, Benzolsulfonyloxy oder Benzolsulfonyloxy, das mit einem oder mehreren Substituenten substituiert ist, der aus der Gruppe ausgewählt ist bestehend aus Halogen, C₁-C₆-Alkyl und C₁-C₆₋Alkoxy;
wobei das Verfahren zur Herstellung die Umsetzung der Verbindung mit einer Struktur der folgenden Formel umfasst 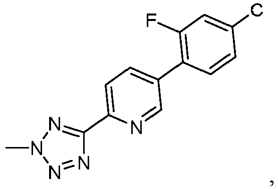
wobei C Hydroxyl oder Amino ist;
1) mit Sulfonylchlorid Verbindungen, wenn C Hydroxyl ist, um die Verbindung mit einer Struktur der folgenden Formel herzustellen 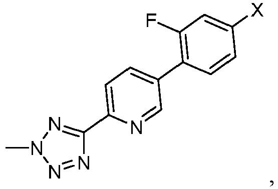 wobei X Sulfonyloxy ist, wie Trifluormethansulfonyloxy, Methansulfonyloxy, Benzolsulfonyloxy oder Benzolsulfonyloxy, das mit einem oder mehreren Substituenten substituiert ist, und wobei der Substituent ausgewählt ist aus der Gruppe bestehend aus Halogen, C₁-C₆-Alkyl und C₁-C₆-Alkoxy; oder
2) wenn C Amino ist, zur Herstellung von DiazoniumsalzVerbindungen in Gegenwart von Natriumnitrit, die dann mit Halogen Ionen substituiert werden, um die Verbindung mit einer Struktur der folgenden Formel herzustellen 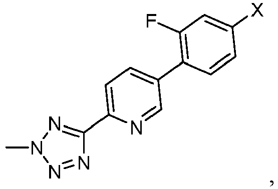 wobei X Chlor, Brom oder Jod ist.

7. Ein Verfahren zur Herstellung der Verbindung der folgenden Formel, 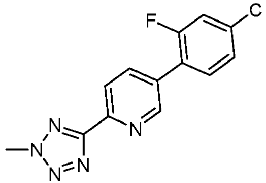 umfassend die Umsetzung der Verbindung mit einer Struktur der folgenden Formel 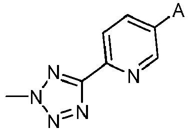 mit der Verbindung mit einer Struktur der folgenden Formel 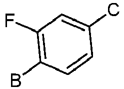 unter der Katalyse von Palladiumkatalysatoren durch eine gekoppelte Reaktion; wobei C Hydroxyl oder Amino ist; eines von A und B Halogen ist, wie Chlor, Brom oder Jod, oder Sulfonyloxy, wie Trifluormethansulfonyloxy, Methansulfonyloxy, Benzolsulfonyloxy oder Benzolsulfonyloxy, das mit einem oder mehreren Substituenten substituiert ist, und der Substituent ausgewählt ist aus der Gruppe bestehend aus Halogen, C₁-C₆-Alkyl und C₁-C₆-Alkoxy; bevorzugt ist einer der Substituenten A und B Chlor, Brom und Jod ist; noch bevorzugter ist einer der Substituenten A und B Brom oder Jod und der andere BF₃ oder BR₂R₃ ist, wobei R₂ und R₃ unabhängig voneinander ausgewählt sind aus der Gruppe bestehend aus OH und einwertigem C₁-C₆-Alkohol und C₁-C₆-Diol oder einwertigem C₁-C₆-Alkohol und C₁-C₆-Diol, die durch Halogen substituiert sind, und wobei R₂ und R₃ einen Ring bilden können; bevorzugt ist BR₂R₃ B(OH)₂ oder 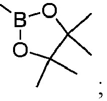 bevorzugt ist B Halogen, wie Chlor, Brom oder Jod, oder Sulfonyloxy, wie Trifluormethansulfonyloxy, Methansulfonyloxy, Benzolsulfonyloxy oder Benzolsulfonyloxy, das mit einem oder mehreren Substituenten substituiert ist, und der Substituent aus der Gruppe ausgewählt ist bestehend aus Halogen, C₁-C₆-Alkyl und C₁-C₆-Alkoxy; bevorzugt ist B Chlor, Brom oder Jod; noch bevorzugter ist B Brom oder Jod,
und A ist BF₃ oder BR₂R₃, wobei R₂ und R₃ unabhängig voneinander ausgewählt werden können aus OH und einwertigem C₁-C₆-Alkohol und C₁-C₆-Diol oder einwertigem C₁-C₆-Alkohol und C₁-C₆-Diol, die durch Halogen substituiert sind, und wobei R₂ und R₃ einen Ring bilden können;
und wobei bevorzugt BR₂R₃ B(OH)₂ oder 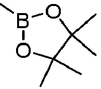 ist; oder bevorzugt ist A Halogen, wie Chlor, Brom oder Jod, oder Sulfonyloxy, wie Trifluormethansulfonyloxy, Methansulfonyloxy, Benzolsulfonyloxy oder Benzolsulfonyloxy, das mit einem oder mehreren Substituenten substituiert ist, und der Substituent aus der Gruppe ausgewählt ist bestehend aus Halogen, C₁-C₆-Alkyl und C₁-C₆-Alkoxy; bevorzugt ist A Chlor, Brom und Jod; noch bevorzugter ist A Brom oder Jod, und B ist BF₃ oder BR₂R₃, wobei R₂ und R₃ unabhängig voneinander aus OH und einwertigem C₁-C₆-Alkohol und C₁-C₆-Diol oder einwertigem C₁-C₆-Alkohol und C₁-C₆-Diol, die mit Halogen substituiert sind, ausgewählt werden können, und wobei R₂ und R₃ einen Ring bilden können; und wobei bevorzugt BR₂R₃ B(OH)₂ oder 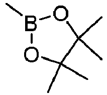 ist.

8. Das Verfahren zur Herstellung nach Anspruch 7, wobei der Palladiumkatalysator Palladiumchlorid, Palladiumacetat, Bis(dibenzylidenaceton)palladium, Tetrakis(triphenylphosphin)palladium, Dichlor[1,1'-bis(diphenylphosphino)ferrocen]palladium, Dichlorbis(tricyclohexylphosphin)palladium oder Dichlorbis(triphenylphosphin)palladium ist.

9. Das Verfahren zur Herstellung nach Anspruch 7 oder 8, wobei das Reaktionslösungsmittel für die gekoppelte Reaktion eines von Wasser, Toluol, Tetrahydrofuran, N,N-Dimethylformamid, Dimethylsulfoxid, Dioxan, Isopropanol, Ethanol oder Acetonitril oder irgendeine Kombination davon und vorzugsweise Toluol, Wasser und Dioxan oder Isopropanol ist und die Reaktionstemperatur 50-120 °C und vorzugsweise 70-100 °C beträgt.

10. Eine Verbindung mit der folgenden chemischen Formel: 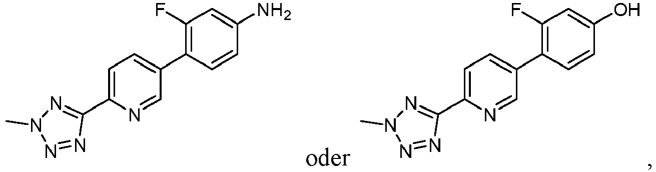 oder der folgenden chemischen Formel: 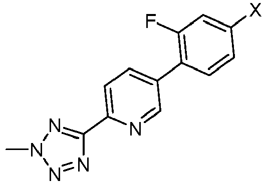 wobei X Chlor, Brom, Jod oder Sulfonyloxy ist, wie Trifluormethansulfonyloxy, Methansulfonyloxy, Benzolsulfonyloxy oder Benzolsulfonyloxy, das mit einem oder mehreren Substituenten substituiert ist, der aus der Gruppe ausgewählt ist bestehend aus Halogen, C₁-C₆-Alkyl und C₁-C₆-Alkoxy; und bevorzugt X Brom oder Jod ist, d.h. die Verbindung der folgenden Formel:

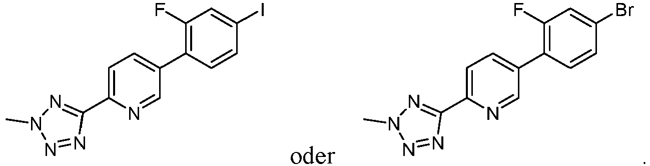

## Revendications

1. Procédé de préparation d'un composé de type tédizolide, de formule donnée ci-dessous : 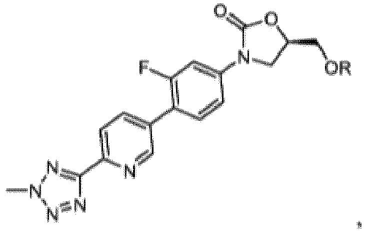 dans laquelle R représente une entité choisie dans l'ensemble formé par un atome d'hydrogène, un groupe de formule 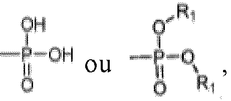 un groupe benzyle, ou un groupe benzyle porteur d'un substituant choisi dans l'ensemble formé par un atome d'halogène et des groupes nitro, alkyle en C₁-C₆ et alcoxy en C₁-C₆, étant entendu que R₁ représente un groupe alkyle en C₁-C₆ ou alkyle en C₁-C₆ porteur d'un ou de substituant(s) halogéno,
lequel procédé de préparation comporte le fait de faire réagir un composé de structure représentée par la formule donnée ci-dessous : 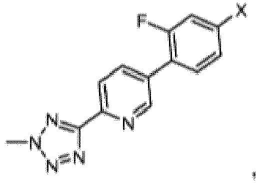 dans laquelle X représente un atome de chlore, de brome ou d'iode ou un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s) choisi(s) dans l'ensemble formé par des atomes d'halogène et des groupes alkyle en C₁-C₆ et alcoxy en C₁-C₆,
avec le composé de structure représentée par la formule donnée ci-dessous : 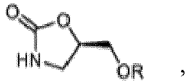 dans laquelle le substituant symbolisé par R est tel qu'il a été défini plus haut,
dans des conditions de catalyse par un catalyseur à base de métal, pour produire le composé de type tédizolide par réaction de couplage.

2. Procédé de préparation conforme à la revendication 1, dans lequel le catalyseur à base de métal est un catalyseur à base de cuivre ou un catalyseur à base de palladium, étant entendu
- que le catalyseur à base de cuivre est de préférence choisi dans l'ensemble consistant en CuI, CuBr, CuCl, CuO, Cu₂O, CuSO₄, Cu(OAc)₂, Cu(OTf)₂ ou du cuivre en poudre, ou mieux encore, est CuI ou Cu(OAc)₂,
- et que le catalyseur à base de palladium est de préférence choisi dans l'ensemble consistant en : chlorure de palladium, acétate de palladium, bis(dibenzylidène-acétone)-palladium, tétrakis(triphényl-phosphine)-palladium, tris(dibenzylidène-acétone)-dipalladium, dichloro[1,1'-bis(diphényl-phosphino)ferrocène]-palladium, dichloro-bis(tricyclohexyl-phosphine)-palladium ou dichloro-bis(triphényl-phosphine)-palladium, ou mieux encore, est du tris(di-benzylidène-acétone)-dipalladium, du chlorure de palladium, ou de l'acétate de palladium.

3. Procédé de préparation conforme à la revendication 1 ou 2, dans lequel la réaction de couplage est réalisée en présence d'une substance alcaline servant de promoteur, laquelle substance alcaline est de préférence choisie dans l'ensemble formé par les suivantes : carbonate de potassium, carbonate de sodium, carbonate de césium, fluorure de césium, acétate de potassium, hydroxyde de sodium, hydroxyde de potassium, phosphate de potassium, ou phosphate de sodium.

4. Procédé de préparation conforme à l'une quelconque des revendications 1 à 3, dans lequel le solvant de réaction utilisé pour la réaction de couplage est du toluène, du chlorobenzène, du tétrahydrofurane, du N,N-diméthyl-formamide, du diméthyl-sulfoxyde, du dioxane, de l'isopropanol, de l'éthanol ou de l'acétonitrile, et de préférence, du N,N-diméthyl-formamide ou du dioxane, et la température de réaction vaut de 60 à 110 °C, et de préférence, de 90 à 110 °C.

5. Procédé de préparation conforme à l'une quelconque des revendications 1 à 4, dans lequel, quand le catalyseur à base de métal est un catalyseur à base de cuivre, ce catalyseur à base de cuivre agit conjointement avec des ligands qui sont choisis dans l'ensemble formé par un ligand de type diamine, un ligand de type dicétone, un ligand de type phénanthroline, un ligand de type acide aminé, et un ligand de type Phos, étant entendu que, de préférence,
- le ligand de type diamine est choisi dans l'ensemble formé par les suivants : 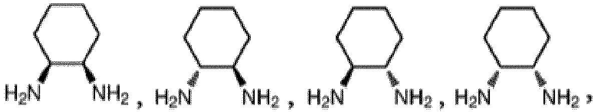 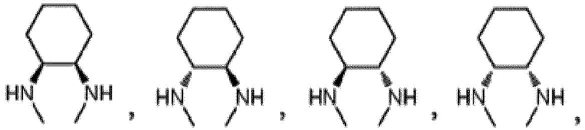 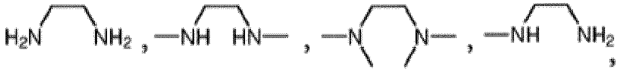 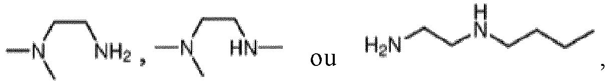
- le ligand de type dicétone est choisi dans l'ensemble formé par les suivants : 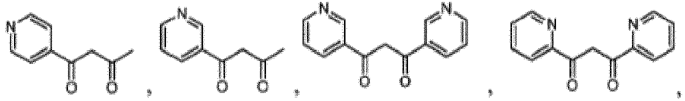 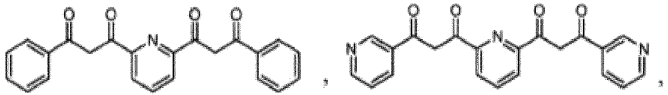 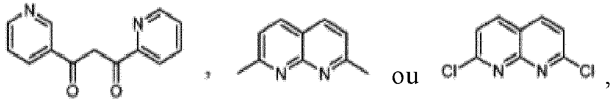
- le ligand de type phénanthroline est choisi dans l'ensemble formé par les suivants : 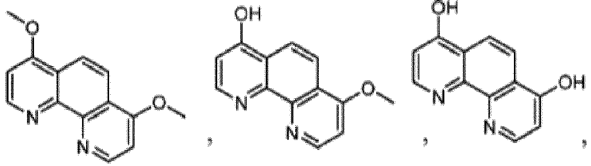 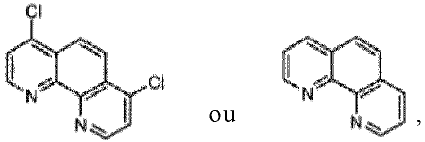
- le ligand de type acide aminé est choisi dans l'ensemble formé par les suivants : 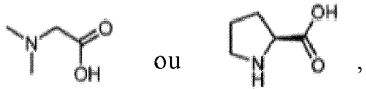
- et le ligand de type Phos est choisi dans l'ensemble formé par les suivants : X-Phos, XantPhos, RuPhos, BrettPhos, SPhos, DavePhos, JohnPhos ou tBuXPhos.

6. Procédé de préparation d'un composé de formule donnée ci-dessous : 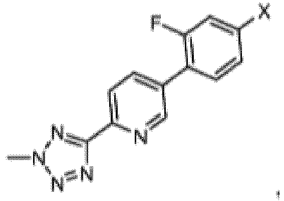 dans laquelle X représente un atome de chlore, de brome ou d'iode ou un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s) choisi(s) dans l'ensemble formé par des atomes d'halogène et des groupes alkyle en C₁-C₆ et alcoxy en C₁-C₆,
lequel procédé de préparation comporte le fait de faire réagir un composé de formule donnée ci-dessous : 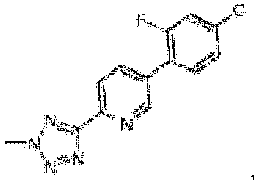 dans laquelle C représente un groupe hydroxyle ou amino,
1) avec des composés de type chlorure de sulfonyle, si C représente un groupe hydroxyle, afin de produire un composé de structure représentée par la formule ci-dessous : 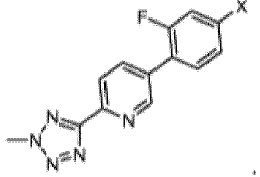 dans laquelle X représente un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s), étant entendu que le substituant est choisi dans l'ensemble formé par des atomes d'halogène et des groupes alkyle en C₁-C₆ et alcoxy en C₁-C₆,
2) ou bien, si C représente un groupe amino, en présence de nitrite de sodium, afin de produire un composé de type sel de diazonium, auquel on fait ensuite subir une substitution avec un ion halogénure, afin de produire un composé de structure représentée par la formule ci-dessous : 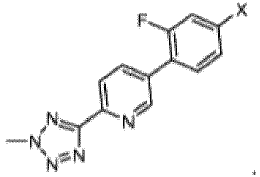 dans laquelle X représente un atome de chlore, de brome ou d'iode.

7. Procédé de préparation d'un composé de formule donnée ci-dessous : 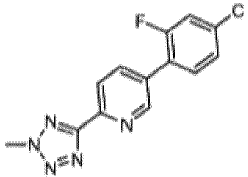 comportant le fait de faire réagir un composé de structure représentée par la formule ci-dessous : 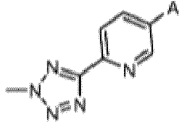 avec un composé de structure représentée par la formule ci-dessous : 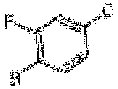 selon une réaction de couplage catalysée par un catalyseur à base de palladium,
dans lesquelles formules
- C représente un groupe hydroxyle ou amino,
- et l'un des symboles A et B représente un atome d'halogène, tel un atome de chlore, de brome ou d'iode, ou un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s), et le substituant est choisi dans l'ensemble formé par des atomes d'halogène et des groupes alkyle en C₁-C₆ et alcoxy en C₁-C₆,
étant entendu que, de préférence, l'un des symboles A et B représente un atome de chlore, de brome ou d'iode, et mieux encore, l'un des symboles A et B représente un atome de brome ou d'iode, et l'autre représente un groupe symbolisé par BF₃ ou BR₂R₃ où R₂ et R₃ représentent des entités choisies indépendamment dans l'ensemble formé par le groupe consistant en hydroxyle, et des restes de monoalcool en C₁-C₆ et de diol en C₁-C₆, ou des restes de monoalcool en C₁-C₆ ou de diol en C₁-C₆ porteur de substituant(s) halogéno, étant entendu que les entités représentées par R₂ et R₃ peuvent former un cycle, et que de préférence, le groupe symbolisé par BR₂R₃ est un groupe de formule B(OH)₂ ou 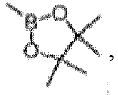
- de préférence, B représente un atome d'halogène, tel un atome de chlore, de brome ou d'iode, ou un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s), et le substituant est choisi dans l'ensemble formé par des atomes d'halogène et des groupes alkyle en C₁-C₆ et alcoxy en C₁-C₆,
étant entendu que, de préférence, B représente un atome de chlore, de brome ou d'iode, et mieux encore, B représente un atome de brome ou d'iode,
et A représente un groupe symbolisé par BF₃ ou BR₂R₃ où R₂ et R₃ représentent des entités choisies indépendamment dans l'ensemble formé par un groupe hydroxyle, des restes de monoalcool en C₁-C₆ et de diol en C₁-C₆, ou des restes de monoalcool en C₁-C₆ et de diol en C₁-C₆ porteur de substituant(s) halogéno, étant entendu que les entités représentées par R₂ et R₃ peuvent former un cycle,
et que de préférence, le groupe symbolisé par BR₂R₃ est un groupe de formule B(OH)₂ ou 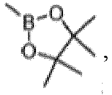
- ou de préférence, A représente un atome d'halogène, tel un atome de chlore, de brome ou d'iode, ou un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s), et le substituant est choisi dans l'ensemble formé par des atomes d'halogène et des groupes alkyle en C₁-C₆ et alcoxy en C₁-C₆ ;
étant entendu que, de préférence, A représente un atome de chlore, de brome ou d'iode, et mieux encore, A représente un atome de brome ou d'iode, et B représente un groupe symbolisé par BF₃ ou BR₂R₃ où R₂ et R₃ représentent des entités choisies indépendamment dans l'ensemble formé par un groupe hydroxyle, des restes de monoalcool en C₁-C₆ et de diol en C₁-C₆, ou des restes de monoalcool en C₁-C₆ et de diol en C₁-C₆ porteur de substituant(s) halogéno, étant entendu que les entités représentées par R₂ et R₃ peuvent former un cycle, et que de préférence, le groupe symbolisé par BR₂R₃ est un groupe de formule B(OH)₂ ou

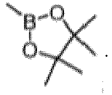

8. Procédé de préparation conforme à la revendication 7, dans lequel le catalyseur à base de palladium est du chlorure de palladium, de l'acétate de palladium, du bis(dibenzylidène-acétone)-palladium, du tétrakis(triphényl-phosphine)-palladium, du dichloro-[1,1'-bis(di-phényl-phosphino)-ferrocène]-palladium, du dichloro-bis(tricyclohexyl-phosphine)-palladium, ou du dichloro-bis(triphényl-phosphine)-palladium.

9. Procédé de préparation conforme à la revendication 7 ou 8, dans lequel le solvant de réaction utilisé pour la réaction de couplage est l'un des suivants : eau, toluène, tétrahydrofurane, N,N-diméthyl-formamide, diméthyl-sulfoxyde, dioxane, isopropanol, éthanol, ou acétonitrile, ou n'importe laquelle de leurs combinaisons, et de préférence, du toluène, de l'eau et du dioxane, ou de l'isopropanol, et la température de réaction vaut de 50 à 120 °C, et de préférence, de 70 à 100 °C.

10. Composé de formule chimique donnée ci-dessous : 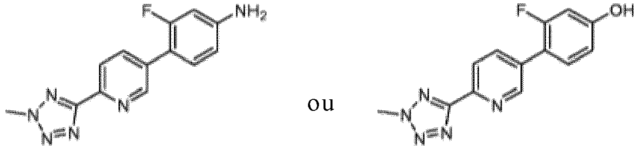 ou de formule chimique suivante : 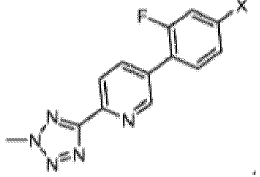 dans laquelle X représente un atome de chlore, de brome ou d'iode, ou un groupe de type sulfonyloxy, tel un groupe trifluorométhane-sulfonyloxy, méthane-sulfonyloxy, benzène-sulfonyloxy, ou benzène-sulfonyloxy porteur d'un ou de plusieurs substituant(s) choisi(s) dans l'ensemble formé par des atomes d'halogène et des groupes alkyle en C₁-C₆ et alcoxy en C₁-C₆, et dans laquelle, de préférence, X représente un atome de brome ou d'iode, soit un composé de formule donnée ci-dessous :
